# EUROPEAN PATENT APPLICATION

(11) **EP 4 691 428 A1**
(43) Date of publication of application: **11.02.2026**
(21) Application number: 24777584.4
(22) Date of filing: 23.02.2024
(51) Int. Cl.: A61F 2/90

(54) **FENESTRATED STENT, COVERED STENT SYSTEM, AND SUTURING METHOD FOR FENESTRATED STENT**

(30) Priority: 30.03.2023 CN 202310327060
(71) Applicant: Hangzhou Endonom Medtech Co., Ltd., Hangzhou, Zhejiang 311217 (CN)
(72) Inventor: WANG, Yongsheng, Hangzhou, Zhejiang 311217 (CN); LI, Anwei, Hangzhou, Zhejiang 311217 (CN); WANG, Lei, Hangzhou, Zhejiang 311217 (CN)
(74) Representative: Mathys & Squire
(86) International application number: PCT/CN2024/078383
(87) International publication number: WO 2024/198794

(57) **Abstract**

A fenestrated stent graft, a stent graft system, and a method for suturing a fenestrated stent graft are provided. The fenestrated stent graft includes a tubular covering membrane and at least one enveloping band. The tubular covering membrane is a tubular structure with openings at two opposite ends, defines a communication cavity, and is provided with at least one fenestration. The at least one enveloping band surrounds the at least one fenestration in a circumferential direction thereof. The enveloping band is at least partially located on an outer circumferential wall of the tubular covering membrane and includes a first region and a second region. The first region is disposed in close contact with and surrounds a corresponding one of the at least one fenestration, the second region is disposed in close contact with and surrounds the first region, and a thickness of the first region is greater than a thickness of the second region. The disclosure has the effect of enhancing the vessel wall apposition between the fenestration and the vessel wall after implantation into a target vessel, thereby addressing the issue of endoleak that tends to occur after implantation of the fenestrated stent graft into the target vessel.

## Description

### CROSS-REFERENCE TO RELATED APPLICATION(S)

This application claims priority to Chinese Patent Application No. 202310327060.3, filed with the Chinese Patent Office on March 30, 2023 and entitled "FENESTRATED STENT GRAFT, STENT GRAFT SYSTEM, AND METHOD FOR SUTURING FENESTRATED STENT GRAFT", which is incorporated herein by reference in its entirety.

### TECHNICAL FIELD

The disclosure relates to the field of medical devices, and in particular, to a fenestrated stent graft, a stent graft system, and a method for suturing a fenestrated stent graft.

### BACKGROUND

Complex abdominal aortic aneurysms (AAAs) generally refer to juxtarenal AAAs (JRAAAs) or AAAs involving branches of visceral arteries. Primary challenges in treating such aneurysms lie in comprehensive evaluation of a proximal anchoring region of an aneurysm sac and proper reconstruction of visceral branch arteries and renal arteries. Currently, treatment of the complex AAAs has entered the era of endovascular interventions. The endovascular interventions primarily involve delivering a vascular stent to a lesion site through a corresponding interventional device and then deploying the vascular stent (the vascular stent can be accommodated within the interventional device in a compressed state and can self-expand to a predetermined shape upon deployment), thereby isolating a vascular dissection entry or an expanded aneurysm sac, reconstructing a true lumen of a vessel, restoring a proper direction of blood flow, and ultimately achieving a therapeutic effect.

The endovascular interventions mainly include parallel stent graft techniques, fenestration techniques, and branch stent graft techniques, which are characterized by minimal invasiveness and rapid recovery. The fenestration technique refers to deployment of a branch stent graft into a target branch artery through a fenestration formed in a main stent graft, which can effectively reconstruct the visceral branch arteries (for example, the celiac trunk artery, superior mesenteric artery, or bilateral renal arteries on the abdominal aorta, or the left subclavian artery, brachiocephalic trunk artery, or left common carotid artery on the aortic arch). However, in the fenestration techniques, at the fenestration of the main stent graft, endoleak may occur due to the poor apposition of the fenestration in the vessel wall. Such poor apposition may result from factors such as high-velocity blood flow impact, improper patient selection, inappropriate device choice, inaccurate deployment or positioning of the fenestrated stent graft, or irregularities of vessel cross-section. More seriously, such poor apposition may cause medical incidents such as paraplegia or death, not only resulting in a failure to achieve the desired therapeutic effect but also causing significant physical and psychological harm to the patient.

### SUMMARY

In order to address the problem of endoleak caused by poor vessel wall apposition of a main stent graft at a fenestration of the main stent graft after implantation into a target vessel, the disclosure provides a fenestrated stent graft. The fenestrated stent graft includes a tubular covering membrane, a support frame, and at least one enveloping band. The tubular covering membrane is a tubular structure with openings at two opposite ends, defines a communication cavity, and is provided with at least one fenestration. The support frame is implemented as multiple support frames that are arranged in an axial direction of the tubular covering membrane. The at least one enveloping band surrounds the at least one fenestration, where for each of the at least one enveloping band, the enveloping band is at least partially located on an outer circumferential wall of the tubular covering membrane and includes a first region and a second region. The first region is disposed in close contact with and surrounds a corresponding one of the at least one fenestration, the second region is disposed in close contact with and surrounds a side of the first region away from the corresponding one of the at least one fenestration, and a thickness of the first region is greater than a thickness of the second region.

In a second aspect, the disclosure further provides a stent graft system. The stent graft system includes the fenestrated stent graft, a bifurcated stent graft, and at least one graft extension. A proximal end of the bifurcated stent graft is fitted with a distal end of the fenestrated stent graft. The bifurcated stent graft includes a proximal tube segment, a first side branch, and a second side branch, the first side branch is fixed to a distal end of the proximal tube segment and in communication with the proximal tube segment, and the second side branch is fixed to the distal end of the proximal tube segment and in communication with the proximal tube segment, such that the proximal tube segment is configured to shunt along the first side branch and the second side branch, and a proximal end of the at least one graft extension is fitted with a distal end of at least one of the first side branch or the second side branch.

In a third aspect, the disclosure further provides a stent graft system. The stent graft system includes the fenestrated stent graft, a bifurcated stent graft, and at least one graft extension. A proximal end of the bifurcated stent graft is fixed to a distal end of the fenestrated stent graft, and the bifurcated stent graft is integrally formed with the fenestrated stent graft. The bifurcated stent graft includes a proximal tube segment, a first side branch, and a second side branch, the first side branch is fixed to a distal end of the proximal tube segment and in communication with the proximal tube segment, and the second side branch is fixed to the distal end of the proximal tube segment and in communication with the proximal tube segment, such that the proximal tube segment is configured to shunt along the first side branch and the second side branch. A proximal end of the at least one graft extension is fixed to a distal end of at least one of the first side branch or the second side branch, and the at least one graft extension is integrally formed with at least one of the first side branch or the second side branch.

In a fourth aspect, the disclosure further provides a stent graft system. The stent graft system includes the fenestrated stent graft and an interventional device for delivering the fenestrated stent graft to a target position. The interventional device includes a tip, a delivery inner shaft fixed to a distal end of the tip, a delivery sheath sleeved over the delivery inner shaft, a delivery handle configured to control axial movement of the delivery sheath, a first pre-embedded guidewire, a second pre-embedded guidewire, and a third pre-embedded guidewire. A gap is defined between the delivery sheath and the delivery inner shaft for accommodating the fenestrated stent graft that is radially compressed. The first pre-embedded guidewire is at least partially located outside the interventional device, at least partially extends through the delivery sheath, and at least partially extends out of a proximal end of the fenestrated stent graft. The tubular covering membrane defines at least one fenestration, where the at least one fenestration includes a second fenestration. For a portion of the first pre-embedded guidewire that is located within the delivery sheath, part of the portion of the first pre-embedded guidewire that is on a distal side of the second fenestration is located outside the fenestrated stent graft, and part of the portion of the first pre-embedded guidewire that is adjacent to the proximal end of the fenestrated stent graft passes through the second fenestration and is located within the communication cavity of the fenestrated stent graft. The fenestrated stent graft further includes at least two embedded branch tubes. Each of the second pre-embedded guidewire and the third pre-embedded guidewire is at least partially located outside the interventional device, the second pre-embedded guidewire at least partially extends through one of the at least two embedded branch tubes, and the third pre-embedded guidewire at least partially extends through another one of the at least two embedded branch tubes.

In a fifth aspect, the disclosure further provides a method for suturing a fenestrated stent graft. The method for suturing the fenestrated stent graft includes the following.

The enveloping band is disposed to surround the at least one fenestration. The enveloping band includes a first side edge and a second side edge opposite the first side edge, and the first side edge is closer to the at least one fenestration than the second side edge. The first side edge is folded to form the first region that has a multilayer membrane structure. The second side edge is made to form the second region that has at least one of a single-layer membrane structure or a multilayer membrane structure. A number of membrane layers of the second region is less than a number of membrane layers of the first region. The first region covers and surrounds at least a portion of the at least one fenestration. The first region is sutured to the fenestrated stent graft through a suture. The second region is sutured to the fenestrated stent graft through a suture.

All the tubular covering membrane, the enveloping band, and the embedded membrane are made of biocompatible fabric, including but not limited to: woven or knitted polyester, such as polyethylene terephthalate, polyethylene glycol terephthalate, polylactide, polyglycolide, and copolymers thereof; fluorinated polymers, such as polytetrafluoroethylene (PTFE), expanded or electrospun PTFE, and polyvinylidene fluoride (PVDF); polysiloxanes, such as polydimethylsiloxane; polyurethanes, such as polyetherurethane, polyurethane urea, polyether polyurethane urea, polyurethanes containing carbonate bonds, and polyurethanes containing siloxane segments wound with nitinol; silicone, ultra-high-molecular-weight polyethylene (UHMWPE), fluorinated ethylene propylene copolymer (FEP), or other suitable materials.

All the support frame, the first support ring, the second support ring, the embedded frame, and the wavy frame are made of elastic materials, such that they are capable of radial compression and deployment, have radial expansion capabilities, can be compressed under external force and either self-expand or be mechanically expanded to restore and maintain their original shape after the removal of the external force. As a result, when implanted into a vessel, these components can conform to the vessel wall by virtue of their radial support force. The elastic materials include, but are not limited to, one or more of nitinol, superelastic nitinol alloys, cobalt-chromium-nickel-molybdenum alloys, copper-based shape memory alloys, iron-based shape memory alloys, medical-grade stainless steel alloys, or various polymers (such as polynorbornene, polyurethane, polylactic acid copolymers, etc.).

In the fenestrated stent graft provided in the disclosure, the thickness of the second region of the enveloping band is less than the thickness of the first region, such that a region of the enveloping band closer to the fenestration is thicker, while a region of the enveloping band farther away from the fenestration is relatively thinner. In a radial direction of the fenestration radiating outward from the center of the fenestration, there is a gradual decrease in in the thicknees of the enveloping band, such that there is a smooth transition in the thickness of the enveloping band. This reduces the height difference between the thickness of the enveloping band and the wire diameter of the support frame, increases both a contact area between the support frame and the enveloping band and a contact area between the support frame and the vessel wall, enhances the wall apposition of the fenestrated stent graft, further reduces the risk of endoleak, and addresses the problem of poor conformity between the fenestration of the fenestrated stent graft and the vessel wall after implantation into the target vessel, which may result in endoleak.

**In** the stent graft system provided in the disclosure, the fenestrated stent graft, the bifurcated stent graft, and the graft extension may be formed separately and modularly assembled, thereby enhancing reconstruction of each branch artery and helping to ensure adequate blood supply to each branch artery.

**In** the stent graft system provided in the disclosure, the fenestrated stent graft, the bifurcated stent graft, and the graft extension may be integrally formed, thereby enhancing reconstruction of each branch artery, helping to ensure adequate blood supply to each branch artery, and contributing to reducing the occurrence of various types of endoleaks, for example, preventing type I or type III endoleaks.

**In** the stent graft system provided in the disclosure, multiple pre-embedded guidewires are provided, which helps to reduce the difficulty of superselective catheterization of visceral arteries, improves the positioning and deployment accuracy of each branch stent graft, thereby helping to prevent displacement of the branch stent grafts, improving the long-term patency of the branch stent grafts, and reducing the reintervention rate.

The method for suturing the fenestrated stent graft provided in the disclosure allows for a smoother transition between the first region and the second region of the enveloping band, thereby increasing the wall apposition of the enveloping band, and helping to avoid the risk of endoleak occurring at the fenestration or the proximal end of the fenestrated stent graft.

### BRIEF DESCRIPTION OF THE DRAWINGS

FIG. 1 is a schematic perspective structural view of a fenestrated stent graft according to a first embodiment.
FIG. 2 is a planar unfolded view of the fenestrated stent graft according to the first embodiment.
FIG. 3 is a schematic structural view illustrating that an enveloping band surrounds and is fixed to a first fenestration according to the first embodiment.
FIG. 4 is a schematic structural view illustrating that an enveloping band surrounds and is fixed to a second fenestration according to the first embodiment.
FIG. 5 is a schematic perspective structural view of the fenestrated stent graft in the first embodiment, viewed from another angle.
FIG. 6 is a schematic perspective structural view illustrating part of a tubular covering membrane and embedded branch tubes in the first embodiment.
FIG. 7 is a partial schematic structural view of the fenestrated stent graft of the first embodiment, where a support frame and part of the tubular covering membrane are omitted.
FIG. 8 is a partial schematic structural view of the fenestrated stent graft of the first embodiment according to another possible implementation, where the support frame and part of the tubular covering membrane are omitted.
FIG. 9 is a schematic view illustrating an application scenario of the fenestrated stent graft of the first embodiment for reconstruction of an abdominal aorta.
FIG. 10 is a schematic view illustrating an application scenario of the fenestrated stent graft of the first embodiment for reconstruction of an aortic arch.
FIG. 11 is another schematic view illustrating an application scenario of the fenestrated stent graft of the first embodiment for reconstruction of an aortic arch.
FIG. 12 is a schematic view illustrating an application scenario of a stent graft system of a second embodiment for reconstruction of an abdominal aorta.
FIG. 13 is a schematic structural view illustrating an application scenario of a stent graft system of a third embodiment for reconstruction of an abdominal aorta.
FIG. 14 is a schematic structural view of a fenestrated stent graft, that is at least partially self-expanded, of a stent graft system of a fourth embodiment.
FIG. 15 is a schematic structural view of the fenestrated stent graft, that is at least partially self-expanded, of the stent graft system of the fourth embodiment, viewed from another angle.
FIG. 16 is a partial schematic view of FIG. 3.
FIG. 17 is a partial schematic view of FIG. 3 according to another embodiment.

Reference numerals are described as follows:
10 - tubular covering membrane; 11 - communication cavity; 12 - branch fenestration; 13 - first tube segment; 14 - second tube segment; 15 - third tube segment; 20 - support frame; 200A - included angle; 20A - first reinforced anti-leakage region; 20B - second reinforced anti-leakage region; 20A1 - density-enhanced region; 20C - anchoring region; 20D - pre-embedded guidewire; 201 - support rod; 202 - crest; 203 - trough; 21 - bare stent; 211 - avoidance region; 212 - bare-end density-enhanced region; 22 - first frame; 221 - support trough; 222 - first support rod; 223 - second support rod; 224 - first connecting rod; 225 - second connecting rod; 226 - first connecting trough; 227 - second connecting trough; 228 - proximal-end density-enhanced region; 23 - second frame; 231 - attaching ring; 232 - third support rod; 233 - fourth support rod; 234 - attaching trough; 235 - third connecting rod; 236 - fourth connecting rod; 237 - third connecting trough; 238 - fourth connecting trough; 239 - reinforced density-enhanced region; 24 - third frame; 25 - fourth frame; 30 - enveloping band; 301 - first region; 302 - second region; 303 - proximal-end support ring; 40 - fenestration; 401 - first fenestration; 402 - second fenestration; 403 - first support ring; 404 - second support ring; 405 - inner edge; 406 - outer edge; 50 - enveloping suture group; 60 - fixation suture group; 70 - suture group; 81 - first anchoring trough; 82 - second anchoring trough; 90 - embedded branch tube; 91 - embedded tube segment; 92 - skirt tube segment; 93 - embedded membrane; 94 - embedded frame; 95 - fixation ring; 96 - reinforcement ring; 100 - clearance region; 101 - first clearance rod; 102 - second clearance rod; 103 - clearance trough; 120 - abdominal aorta; 130 - aneurysm sac; 140 - branch stent graft; 160 - bilateral renal arteries; 170 - superior mesenteric artery; 180 - celiac trunk artery; 190 - aortic arch; 200 - brachiocephalic artery; 210 - left common carotid artery; 220 - left subclavian artery; 230 - bifurcated stent graft; 2301 - proximal tube segment; 2302 - first side branch; 2303 - second side branch; 240 - graft extension; 250 - iliac artery; 260 - tip; 270 - delivery inner shaft; 280 - delivery sheath; 290 - first pre-embedded guidewire; 300 - second pre-embedded guidewire; 310 - third pre-embedded guidewire.

### DETAILED DESCRIPTION

Technical solutions in embodiments of the disclosure are clearly and completely described in the following with reference to the accompanying drawings. Apparently, the described embodiments are merely part of rather than all of the embodiments of the disclosure. All other embodiments obtained by those of ordinary skill in the art based on the embodiments provided herein without creative efforts shall fall within the scope of the disclosure.

In the field of interventional medical devices, a proximal end refers to one end of a stent that is closer to the heart after the stent is deployed for interventional treatment, and a distal end refers to one end of the stent that is farther away from the heart after the stent is deployed for interventional treatment. A direction of a rotation central axis of a cylindrical or tubular object is referred to as an axial direction, and a direction perpendicular to the axial direction is referred to as a radial direction. A circumferential direction refers to a direction along the circumferential, i.e., a direction surrounding the axis of a cylindrical object, a tubular object, or the like (which is perpendicular to both the axial direction and the radius of a cross-section). The "circumferential direction", the "axial direction", and the "radial direction" collectively define three orthogonal directions in a cylindrical coordinate system. A circumferential length refers to an extension length of a structure or component along the circumferential direction of a cylindrical or tubular object. An axial length refers to an extension length of a structure or component along the axial direction of a cylindrical or tubular object. Such illustrations are provided merely for ease of explanation and shall not be constructed as limiting the disclosure. The structures described below refer to the structure of the fenestrated stent graft after expansion.

In interventional therapy, in order to preserve blood flow in branch arteries, a fenestration is typically defined in a main stent graft. The fenestration is directly aligned with an ostium of a branch artery to guide blood flow from the aorta (e.g., the abdominal aorta or aortic arch) into the branch artery, thereby restoring the blood supply to the branch artery. Alternatively, a branch stent graft for reconstruction of a branch artery may be inserted through the fenestration defined in the main stent graft, so as to guide blood flow from the aorta (e.g., the abdominal aorta or aortic arch) into the branch artery, thereby restoring the blood supply to the branch artery. However, due to factors such as the elastic recoil force of the main stent graft, the curved and complex anatomical structure of the vessels, or structural limitations of the main stent graft itself, it is difficult for the fenestration of the main stent graft to tightly conform to the ostium of the branch artery, which results in a gap. Consequently, blood flow from the aorta (e.g., the abdominal aorta or aortic arch) may easily pass through the gap into the aneurysm sac, resulting in endoleak, which is the most common complication after endovascular intervention and has the greatest impact on the effectiveness of the treatment. The endoleak compromises the ability of the main stent graft to isolate the aneurysm sac and thus affects the effectiveness of interventional treatment for aortic aneurysms. In particular, when mural thrombi, calcified plaques, or irregular vascular cross-sections exist near the aortic wall and/or the ostium of the branch artery, the gap between the fenestration and the ostium of the branch artery may be further enlarged, thereby further increasing the risk of endoleak. Alternatively, a gap may be easily formed at the junction between the branch stent graft and the fenestration. Especially when the branch stent graft typically includes a support frame for maintaining the tubular structure of the branch stent graft, blood flow may easily enter the aneurysm sac through a gap formed due to that the support frame protrudes relative to the covering membrane, which may further result in endoleak, for example, endoleak caused by retrograde blood flow.

In order to address the problem of poor vessel wall apposition of the main stent graft at the fenestration of the main stent graft after implantation into a target vessel, which may easily lead to the risk of endoleak, a fenestrated stent graft is provided in a first embodiment of the disclosure. The fenestrated stent graft may be applied within a vessel to perform endovascular exclusion for isolating a lesion region within a lumen. For example, the fenestrated stent graft may be used to isolate an arterial dissection or arterial aneurysm within a lumen of a vessel. It may be understood that the vessel may be the aortic arch, thoracic aorta, or abdominal aorta, etc. One of ordinary skill in the art can appreciate that reference to vessels herein is merely illustrative and shall not be construed as limiting the disclosure. The solutions of the disclosure are applicable to various lumens in the human or animal body, such as lumens of the gastrointestinal tract.

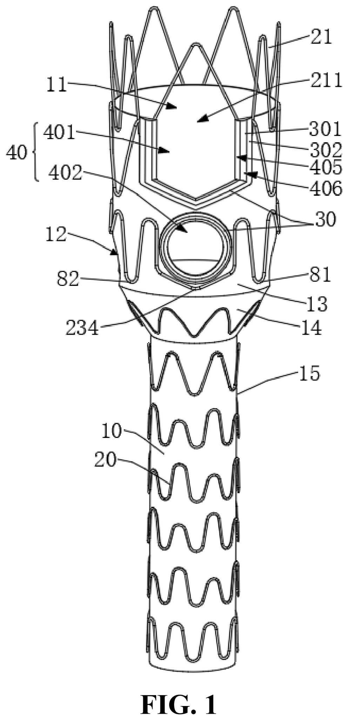

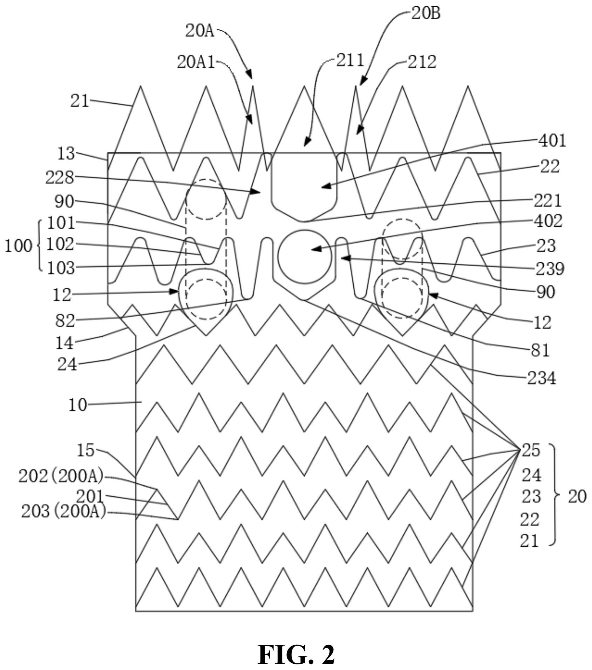

Referring to FIG. 1 and FIG. 2, FIG. 1 is a schematic perspective structural view of the fenestrated stent graft according to the first embodiment, and FIG. 2 is a planar unfolded view of the fenestrated stent graft according to the first embodiment, where an enveloping band 30 is omitted. The fenestrated stent graft includes a tubular covering membrane 10, multiple support frames 20, and at least one enveloping band 30. The tubular covering membrane 10 is a tubular structure with openings at two opposite ends and defines a communication cavity 11 for communication with a target vessel. Each support frame 20 includes multiple support rods 201 connected sequentially at an angle to form a wavy ring structure. Each two adjacent included angles 200A in a circumferential direction of the tubular covering membrane 10 define a crest 202 and a trough 203, and the crest 202 is closer to a proximal end of the tubular covering membrane 10 than the trough 203. The multiple support frames 20 are arranged in an axial direction of the tubular covering membrane 10. The tubular covering membrane 10 defines at least one fenestration 40. At least one enveloping band 30 circumferentially surrounds the at least one fenestration 40. The support frames 20 may be fixed to the tubular covering membrane 10 by suturing, adhering, stamping, attaching, embedding, or thermal pressing.

In some embodiments, the at least one enveloping band 30 may be sutured and secured to the tubular covering membrane 10 through a suture.

In some embodiments, the at least one enveloping band 30 is at least partially located on an outer circumferential wall of the tubular covering membrane 10.

The tubular covering membrane 10 is made of biocompatible fabric, and the enveloping band 30 is made of biocompatible fabric. A fabric density of the biocompatible fabric of the enveloping band 30 is less than a fabric density of the biocompatible fabric of the tubular covering membrane 10, such that the enveloping band 30 is fluffier and softer than the tubular covering membrane 10, facilitating the enveloping band 30 in more effectively filling a gap between the fenestration 40 and a branch stent graft 140 and/or a gap between the fenestration 40 and a vessel wall, thereby enhancing the anti-endoleak effect.

In at least one embodiment, the fenestration 40 includes at least one first fenestration 401 and at least one second fenestration 402, i.e., the tubular covering membrane 10 defines at least two fenestrations 40. The first fenestration 401 and the second fenestration 402 are arranged at intervals in the axial direction of the tubular covering membrane 10. The first fenestration 401 is closer to the proximal end of the tubular covering membrane 10 than the second fenestration 402. The first fenestration 401 penetrates through the proximal end of the tubular covering membrane 10, such that the first fenestration 401 forms an open-loop structure similar to a U-shape.

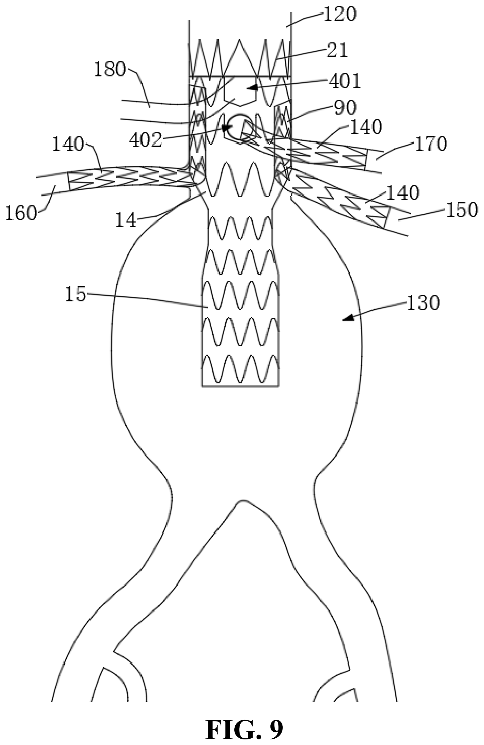

In some embodiments, the first fenestration 401 does not require to be fitted with the branch stent graft 140 through an insertion manner (as illustrated in FIG. 9). For example, the first fenestration 401 may be configured to be in direct communication with an ostium of a celiac trunk artery 180, thereby preventing the tubular covering membrane 10 from blocking blood flow to the celiac trunk artery 180.

In some embodiments, the second fenestration 402 is configured to be fitted with the branch stent graft 140 through an insertion manner for reconstruction of a branch artery, such as a superior mesenteric artery 170, thereby ensuring the patency of the superior mesenteric artery 170.

In at least one embodiment, the fenestrated stent graft further includes a first support ring 403 and a second support ring 404 fixed to the tubular covering membrane 10. The first support ring 403 surrounds the first fenestration 401 to reinforce the shape of the first fenestration 401. The second support ring 404 surrounds the second fenestration 402 to reinforce the shape of the second fenestration 402 and improve the structural stability after the branch stent graft 140 is fitted with the second fenestration 402 through an insertion manner.

An extension tube segment (not illustrated) is further fixed within the second fenestration 402. The extension tube segment is located within the communication cavity 11 of the tubular covering membrane 10, and the second fenestration 402 is in communication with the communication cavity 11 of the tubular covering membrane 10 through the extension tube segment.

In some embodiments, an axial length of the extension tube segment ranges from 2.5 mm to 3.5 mm. At least one wavy frame (not illustrated) is fixed to the extension tube segment. The wavy frame has a waveform structure similar to that of the support frame 20, which will not be repeated herein. The configuration of the extension tube segment helps to increase an anchoring length of the branch stent graft 140, such that the branch stent graft 140 can be more stably inserted into the second fenestration 402, thereby reducing the risk of endoleak after the fenestrated stent graft is fitted with the branch stent graft 140.

In other embodiments, a central axis of the extension tube segment is perpendicular to a central axis of the tubular covering membrane.

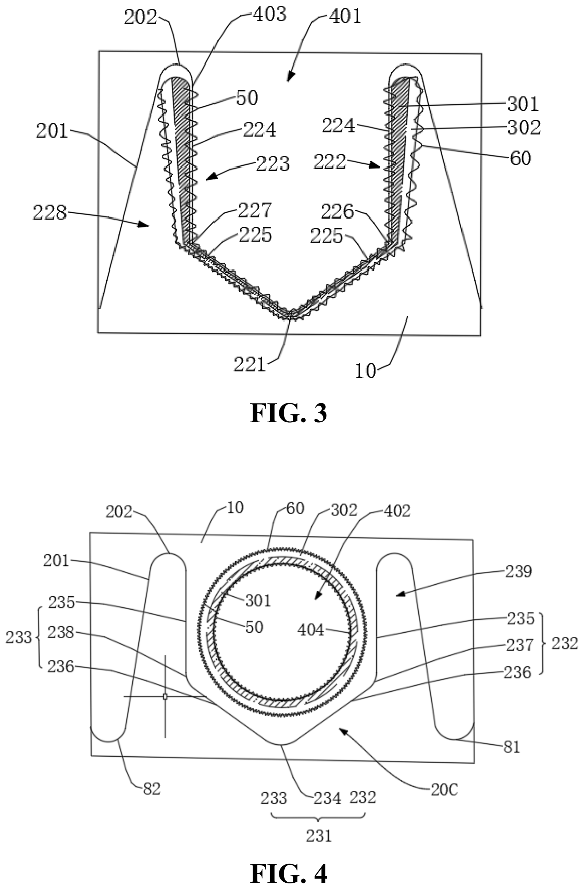

Referring to FIGS. 2 to 4, FIG. 3 is a schematic structural view illustrating that the enveloping band 30 surrounds and is fixed to the first fenestration 401 according to the first embodiment (only the first fenestration 401, the enveloping band 30, part of the support frame 20, and part of the tubular covering membrane 10 are illustrated), and FIG. 4 is a schematic structural view illustrating that the enveloping band 30 surrounds and is fixed to the second fenestration 402 according to the first embodiment (only the second fenestration 402, the enveloping band 30, part of the support frame 20, and part of the tubular covering membrane 10 are illustrated). One enveloping band 30 surrounds and is fixed to the first fenestration 401. Another enveloping band 30 surrounds and is fixed to the second fenestration 402. It may be understood that the shape of the enveloping band 30 varies according to the shape of the fenestration 40, and the first support ring 403 and the second support ring 404 also vary according to the shape of the fenestration 40. For example, in the case where the first fenestration 401 is in a U-shape, the enveloping band 30 and the first support ring 403, which are each circumferentially fixed to the first fenestration 401, are each configured in a U-shaped open-loop structure, thereby preventing the enveloping band 30 and the first support ring 403 from obstructing communication between the first fenestration 401 and an ostium of a target branch artery. In the case where the second fenestration 402 is in a circular shape, an elliptical shape, or other irregular ring-like shapes, the enveloping band 30 and the second support ring 404, which each surround and are fixed to the second fenestration 402, are also configured in a circular shape, elliptical shape, or other irregular closed-loop structure, thereby enabling the enveloping band 30 to more effectively prevent the risk of endoleak.

Referring to FIG. 1, the fenestration 40 includes an inner edge 405 and an outer edge 406. The inner edge 405 of the fenestration 40 is configured to contact an outer circumferential wall of the branch stent graft 140 or an outer circumferential wall of a branch artery. The outer edge 406 of the fenestration 40 is located on the outer circumferential wall of the tubular covering membrane 10. Depending on the distance of different regions of the enveloping band 30 from the center of the fenestration 40, different regions of the enveloping band 30 exhibit different characteristics. Specifically, the enveloping band 30 includes a first region 301 and a second region 302. The first region 301 is closer to the center of the fenestration 40 than the second region 302. In other words, the second region 302 is farther away from the center of the fenestration 40 than the first region 301. The first region 301 is disposed in close contact with and surrounds the fenestration 40. That is, the first region 301 covers and surrounds at least part of the inner edge 405 of the fenestration 40 and at least part of the outer edge 406 of the fenestration 40. The second region 302 is disposed in close contact with and surrounds a side of the first region 301 away from the fenestration 40. The second region 302 surrounds at least part of the outer edge 406 of the fenestration 40. A smooth transition is formed between the first region 301 and the second region 302. A region defined by the enveloping band 30 thereby forms the inner edge 405 and the outer edge 406 of the fenestration 40.

According to exemplary embodiments of the disclosure, in some exemplary embodiments, the first region 301 does not surround at least part of the inner edge 405 of the fenestration 40. That is, the enveloping band 30 is disposed on the outer circumferential wall of the tubular covering membrane 10. The first region 301 covers and surrounds at least part of the outer edge 406 of the fenestration 40, the second region 302 is in close contact with and surrounds the side of the first region 301 away from the fenestration 40, and the second region 302 covers and surrounds at least part of the outer edge 406 of the fenestration 40. A smooth transition is formed between the first region 301 and the second region 302. A region defined by the enveloping band 30 thereby forms the outer edge 406 of the fenestration 40.

A thickness of the first region 301 is greater than a thickness of the second region 302.

The thickness of the first region 301 is greater than a wire diameter of the support frame 20.

The thickness of the second region 302 is less than the wire diameter of the support frame 20.

In a radial direction of the fenestrated stent graft, the thickness of the first region 301 being greater than the wire diameter of the support frame 20 results in the first region 301 located at the outer edge 406 of the fenestration 40 protrudes outward beyond the support frame 20, whereas the thickness of the second region 302 being less than the wire diameter of the support frame 20 results in that the second region 302 is recessed relative to the support frame 20. The thickness of the first region 301 being greater than the wire diameter of the support frame 20 allows the first region 301 of the enveloping band 30 to more effectively filling a gap between the fenestration 40 and a vessel wall and/or a gap between the fenestration 40 and the branch stent graft 140. This configuration provides improved resistance to blood flow impact and reduces the risk of blood entering the aneurysm sac 130 through a gap formed due to that the support frame 20 protrudes relative to the covering membrane 10, which may otherwise result in endoleak, thereby reducing the risk of endoleak after the fenestrated stent graft is implanted in the target vessel.

The thickness of the second region 302 of the enveloping band 30 is less than the wire diameter of the support frame 20, that is, the thickness of the second region 302 of the enveloping band 30 is less than the thickness of the first region 301, such that a region of the enveloping band 30 closer to the center of the fenestration 40 is thicker, while a region of the enveloping band 30 farther away from the center of the fenestration 40 is relatively thinner. In other words, in the radial direction of the fenestration 40 radiating outward from the center of the fenestration 40, there is a gradual decrease in the thickness of the enveloping band 30, such that there is a smooth transition in the thickness of the enveloping band 30. This reduces the height difference between the thickness of the enveloping band 30 and the wire diameter of the support frame 20, increases both a contact area between the support frame 20 and the enveloping band 30 and a contact area between the support frame 20 and the vessel wall, enhances the wall apposition of the fenestrated stent graft, and further reduces the risk of endoleak.

In some embodiments, the wire diameter of the support frame 20 ranges from 0.30 mm to 0.50 mm, the thickness of the first region 301 of the enveloping band 30 ranges from 0.35 mm to 0.60 mm, and the thickness of the second region 302 of the enveloping band 30 ranges from 0.15 mm to 0.28 mm.

In some exemplary embodiments, the first region 301 of the enveloping band 30 is formed by folding a multilayer membrane structure, while the second region 302 of the enveloping band 30 is formed by folding a single-layer membrane structure or a multilayer membrane structure, where the number of membrane layers of the second region 302 is less than a number of membrane layers of the first region 301.

In some exemplary embodiments, referring to FIG. 1 and FIG. 2, in order to enhance the wall apposition of at least part of the support frame around the first fenestration 401 and the second fenestration 402, a central axis of the fenestration 40 is parallel to the central axis of the tubular covering membrane 10 in the axial direction of the tubular covering membrane 10. At least one first reinforced anti-leakage region 20A and at least one second reinforced anti-leakage region 20B are provided on two opposite circumferential sides of the central axis of at least one fenestration 40, respectively. Each of the first reinforced anti-leakage region 20A and the second reinforced anti-leakage region 20B includes at least one density-enhanced region 20A1. Each of the at least one density-enhanced region 20A1 is formed by at least two adjacent support rods 201 of the support frame 20 that are connected to each other. A maximum circumferential length of the two adjacent support rods 201 in the density-enhanced region 20A1 is less than a maximum circumferential length of each two other adjacent support rods 201 of the support frame 20.

In other embodiments, the first reinforced anti-leakage region 20A includes at least two density-enhanced regions 20A1, namely, a proximal-end density-enhanced region 228 and a reinforced density-enhanced region 239 that are arranged in the axial direction of the tubular covering membrane 10.

In other embodiments, the second reinforced anti-leakage region 20B includes at least two density-enhanced regions 20A1, namely, a proximal-end density-enhanced region 228 and a reinforced density-enhanced region 239 that are arranged in the axial direction of the tubular covering membrane 10.

The proximal-end density-enhanced region 228 of the first reinforced anti-leakage region 20A and the proximal-end density-enhanced region 228 of the second reinforced anti-leakage region 20B are located on two opposite circumferential sides of the first fenestration 401, respectively. The reinforced density-enhanced region 239 of the first reinforced anti-leakage region 20A and the reinforced density-enhanced region 239 of the second reinforced anti-leakage region 20B are located on two opposite circumferential sides of the second fenestration 402, respectively.

In this embodiment, in the circumferential direction of the tubular covering membrane 10, the at least one first reinforced anti-leakage region 20A and the at least one second reinforced anti-leakage region 20B are formed on two opposite sides of the central axis of the fenestration 40, respectively. Each of the first reinforced anti-leakage region 20A and the second reinforced anti-leakage region 20B includes a bare-end density-enhanced region 212, the proximal-end density-enhanced region 228, and the reinforced density-enhanced region 239. The bare-end density-enhanced region 212 is formed by at least two adjacent support rods 201 of one support frame 20 that are connected to each other. The proximal-end density-enhanced region 228 is formed by at least two adjacent support rods 201 of another support frame 20 that are connected to each other. The reinforced density-enhanced region 239 is formed by at least two adjacent support rods 201 of another support frame 20 that are connected to each other. The support frame 20 having the bare-end density-enhanced region 212, the support frame 20 having the proximal-end density-enhanced region 228, and the support frame 20 having the reinforced density-enhanced region 239 are arranged adjacent to each other in the axial direction. As such, the bare-end density-enhanced region 212, the proximal-end density-enhanced region 228, and the reinforced density-enhanced region 239 in the first reinforced anti-leakage region 20A are arranged in the axial direction to allow the first reinforced anti-leakage region 20A to extend in the axial direction, and the bare-end density-enhanced region 212, the proximal-end density-enhanced region 228, and the reinforced density-enhanced region 239 in the second reinforced anti-leakage region 20B are arranged in axial direction to allow the second reinforced anti-leakage region 20B to extend in the axial direction.

A maximum circumferential length of two adjacent support rods 201 in the bare-end density-enhanced region 212 is less than a maximum circumferential length of each two other adjacent support rods 201 of the corresponding support frame 20. A maximum circumferential length of two adjacent support rods 201 in the proximal-end density-enhanced region 228 is less than a maximum circumferential length of each two other adjacent support rods 201 of the corresponding support frame 20. A maximum circumferential length of two adjacent support rods 201 in the reinforced density-enhanced region 239 is less than a maximum circumferential length of each two other adjacent support rods 201 of the corresponding support frame 20.

The multiple support frames 20 further include a bare stent 21, a first frame 22, a second frame 23, a third frame 24, and multiple fourth frames 25. The bare stent 21, the first frame 22, the second frame 23, the third frame 24, and the multiple fourth frames 25 are arranged in sequence in the axial direction of the tubular covering membrane 10 from the proximal end of the tubular covering membrane 10 to the distal end of the tubular covering membrane 10. It may be understood that the wire diameter of the support frame 20 refers to the diameter of the support rod 201. The first fenestration 401 is located on a proximal side of the first frame 22 or on the first frame 22. The second fenestration 402 is located between the first frame 22 and the second frame 23.

In at least one embodiment, the bare stent 21 has a closed-loop structure. Troughs 203 of the bare stent 21 are fixed to the tubular covering membrane 10. Crests 202 of the bare stent 21 and at least part of axial portions of the support rods 201 of the bare stent 21 extend beyond the proximal end of the tubular covering membrane 10, that is, the crests 202 of the bare stent 21 and the at least part of the axial portions of the support rods 201 of the bare stent 21 are not covered by the tubular covering membrane 10. In other words, in an axial direction of each support rod 201 of the bare stent 21, at least part of axial portions of each support rod 201 is not covered by the tubular covering membrane 10, and the crests 202 of the bare stent 21 are not covered by the proximal end of the tubular covering membrane 10.

In the circumferential direction of the tubular covering membrane 10, for the bare stent 21, a region defined by two adjacent support rods 201 spans the first fenestration 401 to form an avoidance region 211. Distal ends of the two adjacent support rods 201 in the avoidance region 211 are located on two opposite circumferential sides of the first fenestration 401 in the circumferential direction of the tubular covering membrane 10, respectively, thereby facilitating avoidance of interference by the bare stent 21 with blood flow that enters a branch artery (e.g., the celiac trunk artery 180) through the ostium of the branch artery for reconstruction of a branch artery.

In other embodiments, the bare stent 21 further includes multiple barbs. The multiple barbs are disposed on at least one of each crest 202, each trough 203, and/or each support rod 201 of the bare stent 21, respectively, such that the fenestrated stent graft can be more stably supported within the target vessel.

In the circumferential direction of the tubular covering membrane 10, at least two bare-end density-enhanced regions 212 are formed on two opposite sides of the avoidance region 211, respectively. Each bare-end density-enhanced region 212 is formed by two adjacent support rods 201 of the bare stent 21 that are connected to each other. The two adjacent support rods 201 in the bare-end density-enhanced region 212 are adjacent to the two adjacent support rods 201 in the avoidance region 211 in the circumferential direction of the tubular covering membrane 10.

In some embodiments, a circumferential length between the distal ends of the two adjacent support rods 201 in the bare-end density-enhanced region 212 is less than a circumferential length between distal ends of the two adjacent support rods 201 in the avoidance region 211, and is also less than a circumferential length between distal ends of two adjacent support rods 201 forming each of other crests 202 of the bare stent 21. A circumferential length between the two adjacent support rods 201 in the avoidance region 211 is greater than a circumferential length between two adjacent support rods 201 forming each of other crests 202 of the bare stent 21.

In at least one embodiment, in the circumferential direction of the tubular covering membrane 10, the circumferential length between the distal ends of the two adjacent support rods 201 in the avoidance region 211 accounts for 17% to 24% of a circumferential length of the proximal end of the tubular covering membrane 10, and is no less than 12 mm.

The circumferential length between the distal ends of the two support rods 201 in the bare-end density-enhanced region 212 accounts for 5% to 10% of the circumferential length of the proximal end of the tubular covering membrane 10. The circumferential length between the distal ends of the two adjacent support rods 201 forming each of the other crests 202 accounts for 16% to 20% of the circumferential length of the proximal end of the tubular covering membrane 10. The configuration of the bare-end density-enhanced region 212 enhances the wall apposition and radial support force between the bare stent 21 and the vessel wall.

It may be understood that, in the circumferential direction of the tubular covering membrane 10, the number of bare-end density-enhanced regions 212 on the two opposite sides of the avoidance region 211 may be one, two, three, four, or more, which is not limited herein.

In at least one embodiment, referring to FIG. 2 and FIG. 3, a first support ring 403 is formed by two adjacent support rods 201 of the first frame 22, that is, the first support ring 403 is formed by enclosing two adjacent support rods 201. For ease of distinction, the two support rods 201 forming the first support ring 403 are referred to as a first support rod 222 and a second support rod 223. A distal end of the first support rod 222 and a distal end of the second support rod 223 are connected to each other at an angle to form a trough 203, which is referred to as a support trough 221. The first support rod 222 include one first connecting rod 224 and one second connecting rod 225 connected to each other in the axial direction. The second support rod 223 include one first connecting rod 224 and one second connecting rod 225 connected to each other in the axial direction.

A first connecting trough 226 is formed by the distal end of the first connecting rod 224 of the first support rod 222 and a proximal end of the second connecting rod 225 of the first support rod 222 that are connected to each other at an angle. The proximal end of the first connecting rod 224 of the first support rod 222 serves as the proximal end of the first support rod 222, and the distal end of the second connecting rod 225 of the first support rod 222 serves as the distal end of the first support rod 222.

A second connecting trough 227 is formed by the distal end of the first connecting rod 224 of the second support rod 223 and the proximal end of the second connecting rod 225 of the second support rod 223 that are connected to each other at an angle. The proximal end of the first connecting rod 224 of the second support rod 223 serves as the proximal end of the second support rod 223, and the distal end of the second connecting rod 225 of the second support rod 223 serves as the distal end of the second support rod 223. Both the first connecting trough 226 and the second connecting trough 227 are closer to the proximal end of the tubular covering membrane 10 than other troughs 203 of the first frame 22. The formation of the first connecting trough 226 and the second connecting trough 227 facilitates alignment of the first fenestration 401 with an ostium of a branch artery, and also improves the wall apposition and structural stability of the first fenestration 401.

In the circumferential direction of the tubular covering membrane 10, at least two proximal-end density-enhanced regions 228 are formed on two opposite sides of the first fenestration 401, respectively. That is, at least one proximal-end density-enhanced region 228 is located on each of two opposite circumferential sides of the support trough 221. Each proximal-end density-enhanced region 228 is formed by two adjacent support rods 201 of the first frame 22 that are connected to each other, and the two support rods 201 in the proximal-end density-enhanced region 228 are connected to each other at an angle to form one crest 202 of the first frame 22. The said crest 202 in the proximal-end density-enhanced region 228 and the support trough 221 form two adjacent angles 200A in the circumferential direction of the tubular covering membrane 10.

One of the two support rods 201 in the proximal-end density-enhanced region 228 of the first reinforced anti-leakage region 20A serves as the first support rod 222 (i.e., one support rod 201 in the proximal-end density-enhanced region 228 of the first reinforced anti-leakage region 20A is shared with the first support rod 222 of the proximal-end support ring 303; that is, the two refer to the same support rod 201). One of the two support rods 201 in the proximal-end density-enhanced region 228 of the second reinforced anti-leakage region 20B serves as the second support rod 223 (i.e., one support rod 201 in the proximal-end density-enhanced region 228 of the second reinforced anti-leakage region 20B is shared with the second support rod 223 of the proximal-end support ring 303; that is, the two refer to the same support rod 201).

In other words, the crest 202 in the proximal-end density-enhanced region 228 of the first reinforced anti-leakage region 20A is formed by the proximal end of the first connecting rod 224 of the first support rod 222 and a proximal end of another support rod 201 in the proximal-end density-enhanced region 228 of the first reinforced anti-leakage region 20A that are connected to each other. The crest 202 in the proximal-end density-enhanced region 228 of the second reinforced anti-leakage region 20B is formed by the proximal end of the first connecting rod 224 of the second support rod 223 and a proximal end of another support rod 201 in the proximal-end density-enhanced region 228 of the second reinforced anti-leakage region 20B that are connected to each other.

A circumferential length from a distal end of one support rod 201 in the proximal-end density-enhanced region 228 of the first reinforced anti-leakage region 20A, that is away from the first fenestration 401, to an extension line of the first connecting rod 224 of the first support rod 222, and a circumferential length from a distal end of one support rod 201 in the proximal-end density-enhanced region 228 of the second reinforced anti-leakage region 20B, that is away from the first fenestration 401, to an extension line of the first connecting rod 224 of the second support rod 223, are both less than the circumferential length between the distal ends of the two adjacent support rods 201 forming each of the other crests 202 of the first frame 22. The configuration of the proximal-end density-enhanced region 228 enhances the wall apposition between the first fenestration 401 and the vessel wall.

In at least one embodiment, referring to FIG. 2 and FIG. 4, two adjacent support rods 201 of the second frame 23 form an attaching ring 231. The two adjacent support rods 201 of the attaching ring 231 are referred to as a third support rod 232 and a fourth support rod 233, respectively. A trough 203 formed by a distal end of the third support rod 232 and a distal end of the fourth support rod 233 that are connected to each other is referred to as an attaching trough 234.

Each of the third support rod 232 and the fourth support rod 233 includes a third connecting rod 235 and a fourth connecting rod 236 that are connected to each other in the axial direction. For the third support rod 232, a distal end of the third connecting rod 235 and a proximal end of the fourth connecting rod 236 are connected to each other at an angle to form a third connecting trough 237. A proximal end of the third connecting rod 235 serves as a proximal end of the third support rod 232, and a distal end of the fourth connecting rod 236 serves as the distal end of the third support rod 232.

For the fourth support rod 233, a distal end of the third connecting rod 235 and a proximal end of the fourth connecting rod 236 are connected to each other at an angle to form a fourth connecting trough 238. A proximal end of the third connecting rod 235 serves as a proximal end of the fourth support rod 233, and a distal end of the fourth connecting rod 236 serves as the distal end of the fourth support rod 233. The second support ring 404 is located on a proximal side of the attaching ring 231, such that the attaching ring 231 forms a substantial open-loop structure that covers and surrounds at least part of the second support ring 404, facilitating enhancement of the wall apposition of the second support ring 404 and enhancement of the anti-endoleak effect. The enveloping band 30 on the second fenestration 402 is substantially disposed between the attaching ring 231 and the second support ring 404, further enhancing the wall apposition of the second fenestration 402, and thus enhancing the anti-endoleak effect of the second fenestration 402.

At least one anchoring region 20C is formed on a distal side of the second fenestration 402. Specifically, in the circumferential direction of the tubular covering membrane 10, the attaching trough 234 and at least two troughs 203 adjacent to two opposite sides of the attaching trough 234 are farther away from the proximal end of the tubular covering membrane 10 than other troughs 203 of the second frame 23. For ease of distinction, these at least two troughs 203 adjacent to the two opposite sides of the attaching trough 234 are referred to as a first anchoring trough 81 and a second anchoring trough 82.

In the circumferential direction of the tubular covering membrane 10, the first anchoring trough 81 and the second anchoring trough 82 are located on the two opposite sides of the attaching trough 234, respectively. Two adjacent support rods 201 forming the first anchoring trough 81, the two adjacent support rods 201 forming the second anchoring trough 82, and two adjacent support rods 201 forming the attaching trough 234 each have a longer axial length than other two adjacent support rods 201 forming each of the other troughs 203 of the second frame 23. Moreover, the first anchoring trough 81, the second anchoring trough 82, and the attaching trough 234 are all located farther away from the proximal end of the tubular covering membrane 10 than the other troughs 203 of the second frame 23, thereby defining the anchoring region 20C.

Distances from distal ends of the two adjacent support rods 201 forming the first anchoring trough 81, distal ends of the two adjacent support rods 201 forming the second anchoring trough 82, and distal ends of the two adjacent support rods 201 forming the attaching trough 234 to distal ends of the support rods 201 forming the other troughs 203 of the second frame 23 cooperatively define an axial length of the anchoring region 20C.

In some embodiments, the axial length of the anchoring region 20C ranges from 3 mm to 5 mm. The configuration of the anchoring region 20C enhances the wall apposition of the support frame 20 around the second fenestration 402 and further enhances the anti-endoleak effect.

It may be understood that the number of the anchoring troughs 203 located on the two opposite sides of the attaching trough 234 in the circumferential direction of the tubular covering membrane 10 may be one, two, three, four, or more, which is not limited herein.

In at least one embodiment, in the circumferential direction of the tubular covering membrane 10, at least two reinforced density-enhanced regions 239 are formed on two opposite sides of the attaching trough 234, respectively. Each reinforced density-enhanced region 239 is formed by two adjacent support rods 201 of the second frame 23 that are connected to each other. Two support rods 201 of the reinforced density-enhanced region 239 are connected to each other at an angle to form one crest 202 of the second frame 23. The two adjacent support rods 201 of the reinforced density-enhanced region 239 and support rods 201 of the second support ring 404 are arranged adjacent to each other in the circumferential direction of the tubular covering membrane 10.

The crest 202 in the reinforced density-enhanced region 239 is formed by two adjacent support rods 201 that are connected to each other at an included angle 200A in the circumferential direction of the tubular covering membrane 10. The attaching trough 234 is formed by another two adjacent support rods 201 that are connected to each other at an included angle 200A in the circumferential direction of the tubular covering membrane 10. One support rod 201 in the reinforced density-enhanced region 239 of the first reinforced anti-leakage region 20A serves as the third support rod 232 (i.e., the one support rod 201 in the reinforced density-enhanced region 239 of the first reinforced anti-leakage region 20A is shared with the third support rod 232 of the attaching ring 231; that is, the two refer to the same support rod 201). The other support rod 201 in the reinforced density-enhanced region 239 of the second reinforced anti-leakage region 20B serves as the fourth support rod 233 (i.e., the other support rod 201 in the reinforced density-enhanced region 239 of the second reinforced anti-leakage region 20B is shared with the fourth support rod 233 of the attaching ring 231; that is, the two are the same support rod 201).

In other words, the crest 202 in the reinforced density-enhanced region 239 of the first reinforced anti-leakage region 20A is formed by the proximal end of the third connecting rod 235 of the third support rod 232 and a proximal end of the other support rod 201 in the reinforced density-enhanced region 239 of the first reinforced anti-leakage region 20A that are connected to each other. The crest 202 in the reinforced density-enhanced region 239 of the second reinforced anti-leakage region 20B is formed by the proximal end of the third connecting rod 235 of the fourth support rod 233 and a proximal end of the other support rod 201 in the reinforced density-enhanced region 239 of the second reinforced anti-leakage region 20B that are connected to each other.

A circumferential length from a distal end of one support rod 201 in the reinforced density-enhanced region 239 of the first reinforced anti-leakage region 20A, that is away from the second fenestration 402, to an extension line of the third connecting rod 235 of the third support rod 232, and a circumferential length from a distal end of one support rod 201 in the reinforced density-enhanced region 239 of the second reinforced anti-leakage region 20B, that is away from the second fenestration 402, to an extension line of the third connecting rod 235 of the fourth support rod 233, are both less than a circumferential length between distal ends of two adjacent support rods 201 forming each of other crests 202 of the second frame 23. The configuration of the reinforced density-enhanced region 239 enhances the wall apposition between the second fenestration 402 and the vessel wall.

The bare-end density-enhanced region 212, the proximal-end density-enhanced region 228, and the reinforced density-enhanced region 239 of the first reinforced anti-leakage region 20A are arranged adjacent to each other in the axial direction, such that the first reinforced anti-leakage region 20A substantially axially extends. The bare-end density-enhanced region 212, the proximal-end density-enhanced region 228, and the reinforced density-enhanced region 239 of the second reinforced anti-leakage region 20B are arranged adjacent to each other in the axial direction, such that the second reinforced anti-leakage region 20B substantially axially extends. The first reinforced anti-leakage region 20A and the second reinforced anti-leakage region 20B, which are respectively located on two opposite circumferential sides of the fenestration 40, cooperatively enhance the wall apposition of at least part of the support frame 20 around the first fenestration 401 and the second fenestration 402, thereby more efficiently enhancing the anti-endoleak effect of the first fenestration 401 and the second fenestration 402.

It may be understood that the substantially axial extension of the first reinforced anti-leakage region 20A is not limited to an axial alignment of the crest 202 in the bare-end density-enhanced region 212, the crest 202 in the proximal-end density-enhanced region 228, and the crest 202 in the reinforced density-enhanced region 239 of the first reinforced anti-leakage region 20A. The crest 202 in the bare-end density-enhanced region 212, the crest 202 in the proximal-end density-enhanced region 228, and the crest 202 in the reinforced density-enhanced region 239 of the first reinforced anti-leakage region 20A may deviate to some extent in the axial direction. The substantially axial extension of the first reinforced anti-leakage region 20A may be understood to mean that the crests 202 in the bare-end density-enhanced region 212, the crest 202 in the proximal-end density-enhanced region 228, and the crest 202 in the reinforced density-enhanced region 239 of the first reinforced anti-leakage region 20A are positioned circumferentially adjacent to the fenestration.

It may be understood that the substantially axial extension of the second reinforced anti-leakage region 20A is not limited to an axial alignment of the crest 202 in the bare-end density-enhanced region 212, the crest 202 in the proximal-end density-enhanced region 228, and the crest 202 in the reinforced density-enhanced region 239 of the second reinforced anti-leakage region 20A. The crest 202 in the bare-end density-enhanced region 212, the crest 202 in the proximal-end density-enhanced region 228, and the crest 202 in the reinforced density-enhanced region 239 of the second reinforced anti-leakage region 20A may deviate to some extent in the axial direction. The substantially axial extension of the second reinforced anti-leakage region 20A may be understood to mean that the crest 202 in the bare-end density-enhanced region 212, the crest 202 in the proximal-end density-enhanced region 228, and the crest 202 in the reinforced density-enhanced region 239 of the second reinforced anti-leakage region 20A are positioned circumferentially closer to the fenestration.

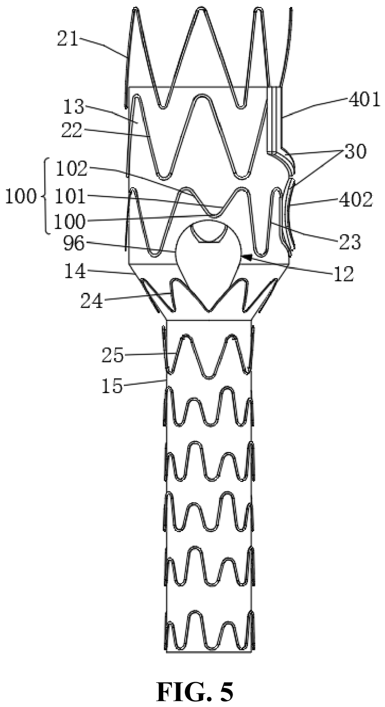

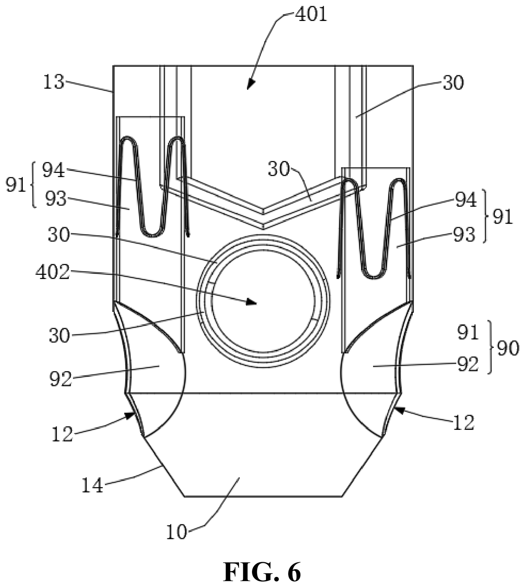

Referring to FIGS. 2, 5, and 6, FIG. 5 is a schematic perspective structural view of the fenestrated stent graft in the first embodiment, viewed from another angle, and FIG. 6 is a schematic perspective structural view illustrating part of a tubular covering membrane and embedded branch tubes in the first embodiment, where the support frame 20 of the fenestrated graft is omitted.

In at least one embodiment, the fenestrated stent graft includes at least two embedded branch tubes 90. The at least two embedded branch tubes 90 are located on two opposite sides of the fenestration 40 in the circumferential direction of the tubular covering membrane 10, respectively. The embedded branch tubes 90 is configured to be fitted with the branch stent graft 140 for reconstruction of a branch artery (for example, bilateral renal arteries 160). The tubular covering membrane 10 further defines at least two branch fenestrations 12. The at least two embedded branch tubes 90 are fixed to the at least two branch fenestrations 12, respectively. The branch fenestration is in communication with the communication cavity 11 of the tubular covering membrane 10 through a corresponding embedded branch tube 90.

The branch fenestration 12 is located between the second frame 23 and the third frame 24.

In other embodiments, at least one branch fenestration 12 is provided with one enveloping band 30.

In at least one embodiment, at least one clearance region 100 is provided on each of two opposite sides of the second fenestration 402 in the circumferential direction of the tubular covering membrane 10, i.e., two clearance regions 100 are located on two opposite sides of the anchoring region 20C, respectively.

In the circumferential direction of the tubular covering membrane 10, at least two clearance regions 100 are located on two opposite sides of the two reinforced density-enhanced regions 239, respectively. In other words, at least one reinforced density-enhanced region 239 is located between the clearance region 100 and the anchoring region 20C. The clearance region 100 is formed on the second frame 23, and a distal end of the clearance region 100 is closer to the proximal end of the tubular covering membrane 10 than a distal end of each of other regions of the second frame 23. An axial length of the clearance region 100 is less than an axial length of each of other regions of the second frame 23.

The clearance region 100 is formed by at least two adjacent support rods 201. For ease of distinction, the at least two support rods 201 in the clearance region 100 are referred to as a first clearance rod 101 and a second clearance rod 102, respectively. A distal end of the first clearance rod 101 and a distal end of the second clearance rod 102 are connected to each other at an angle to form a clearance trough 103. A proximal end of the first clearance rod 101 is connected to a proximal end of an adjacent support rod 201 at an angle to form one crest 202 of the second frame 23, and a proximal end of the second clearance rod 102 is connected to a proximal end of an adjacent support rod 201 at an angle to form another crest 202 of the second frame 23.

A length of each of the two adjacent support rods 201 in the clearance region 100 in the axial direction of the tubular covering membrane 10 is less than a length of each of other support rods 201 of the second frame 23 in the axial direction of the tubular covering membrane 10, such that the clearance trough 103 is closer to the proximal end of the tubular covering membrane 10 than each of other troughs 203 of the second frame 23.

The branch fenestration 12 is located between the clearance trough 103 and a trough 203 of the third frame 24 adjacent to the clearance trough 103 in the axial direction of the tubular covering membrane 10.

Compared to the case where the branch fenestration 12 is disposed on a distal side of the crest 202 of the second frame 23, setting the clearance region 100 to enable the branch fenestration 12 to be on a distal side of the clearance trough 103 in the clearance region 100, which is beneficial for avoiding deformation of the branch fenestration 12 from being restricted by the crest 202 of the second frame 23. This configuration provides greater space for deformation of the branch fenestration 12 (e.g., displacement or shape recovery of the branch fenestration 12 in the axial direction, the circumferential direction, and/or other directions of the tubular covering membrane 10), which is beneficial for avoiding compression of the branch stent graft 140 and enhancing the assemblability of the fenestrated stent graft within a delivery sheath (the delivery sheath being an interventional device for delivering the fenestrated stent graft in a radially compressed state to a lesion region of a target vessel), thereby facilitating assembly of the fenestrated stent graft into the delivery sheath.

It may be understood that distances from distal ends of the two adjacent support rods forming the first anchoring trough 81, distal ends of the two adjacent support rods forming the second anchoring trough 82, and distal ends of the two support rods forming the attaching trough 234 to distal ends of the support rods forming other troughs of the second frame 23 (other than the clearance trough 103) cooperatively define the axial length of the anchoring region 20C.

In at least one embodiment, the tubular covering membrane 10 includes a first tube segment 13, a second tube segment 14, and a third tube segment 15 that are sequentially connected in the axial direction of the tubular covering membrane 10. The first tube segment 13 and the third tube segment 15 may be of a constant-diameter structure. The third tube segment 15 may be of at least one of a substantially constant-diameter structure or a non-constant-diameter structure. The second tube segment 14 be of a substantially tapered, non-equal-diameter structure. A diameter of the second tube segment 14 gradually decreases in a direction from the proximal end of the tubular covering membrane 10 to the distal end of the tubular covering membrane 10. A diameter of the first tube segment 13 connected to a proximal end of the second tube segment 14 is greater than a diameter of the third tube segment 15 connected to a distal end of the second tube segment 14.

It may be understood that each of the first tube segment 13 and the second tube segment 14 may be of a constant-diameter structure relative to the third tube segment 15. In actual manufacturing processes, a tolerance of ±10% is allowed. Alternatively, each of the first tube segment 13 and the second tube segment 14 may have a small degree of taper, such that each of the first tube segment 13 and the second tube segment 14 may not be of a perfectly constant-diameter structure. The third tube segment 15 may be of at least one of a substantially constant-diameter structure or a non-constant-diameter structure. For example, a proximal end of the third tube segment 15 may be tapered, with a gradual increase in the diameter of the proximal end of the third tube segment 15 from the proximal end to the distal end, such that a junction between the proximal end of the third tube segment 15 and the distal end of the second tube segment 14 is a location where the radial dimension of the tubular covering membrane 10 is minimized, thereby presenting a dumbbell-shaped profile and further providing more space for movement of the branch stent graft 140. A distal section of the third tube segment 15 is of a substantially constant-diameter structure.

At least part of the first tube segment 13 (e.g., the proximal end of the first tube segment 13) may be deployed into a healthy section of the target vessel, while the second tube segment 14 and the third tube segment 15 may be deployed into the lesion region of the target vessel.

In some embodiments, all the bare stent 21, the first frame 22, and the second frame 23 are located on the first tube segment 13.

In some embodiments, the third frame 24 is located on the second tube segment 14, such that the third frame 24 forms a corresponding tapered structure.

In some embodiments, multiple fourth frames 25 may be located on the third tube segment 15, such that the branch fenestration 12 is located on the first tube segment 13 and the second tube segment 14, i.e., the branch fenestration 12 extends from the first tube segment 13 to the second tube segment 14.

In the axial direction of the tubular covering membrane 10, the first fenestration 401 is located on the proximal side of the first frame 22, and the second fenestration 402 is located between the first frame 22 and the second frame 23.

In some embodiments, at least one fourth frame 25 adjacent to the proximal end of the third tube segment 15 and at least one fourth frame 25 at a distal end of the third tube segment 15 are both of a constant-height-waves structure (that is, a structure where each of the support rods 201 has the same axial length, and all crests 202 are located on a common circumferential horizontal plane, and all troughs 203 are located on another common circumferential horizontal plane), and the fourth frame 25 at the distal end of the third tube segment 15 is fixed to an inner circumferential wall of the tubular covering membrane 10, which helps prevent the fenestrated stent graft from irritating the vessel wall.

In the axial direction of the tubular covering membrane 10, the other fourth frames 25 between the two fourth frames 25 being of the constant-height-waves structure are each of a structure with a combination of high and low waves, i.e., each fourth frame 25 (among the other fourth frames 25 between the two fourth frames 25 being of a constant-height-waves structure in the axial direction of the tubular covering membrane 10) includes both high crests and low crests. The high crests are closer to the proximal end of the tubular covering membrane 10 than the low crests. The high and low crests are alternately arranged in a periodic pattern in the circumferential direction of the tubular covering membrane 10.

In other embodiments, the high and low crests of each fourth frame 25 (among the other fourth frames 25 between the two fourth frames 25 being of the constant-height-waves structure in the axial direction of the tubular covering membrane 10) may be arranged alternately in a non-periodic pattern.

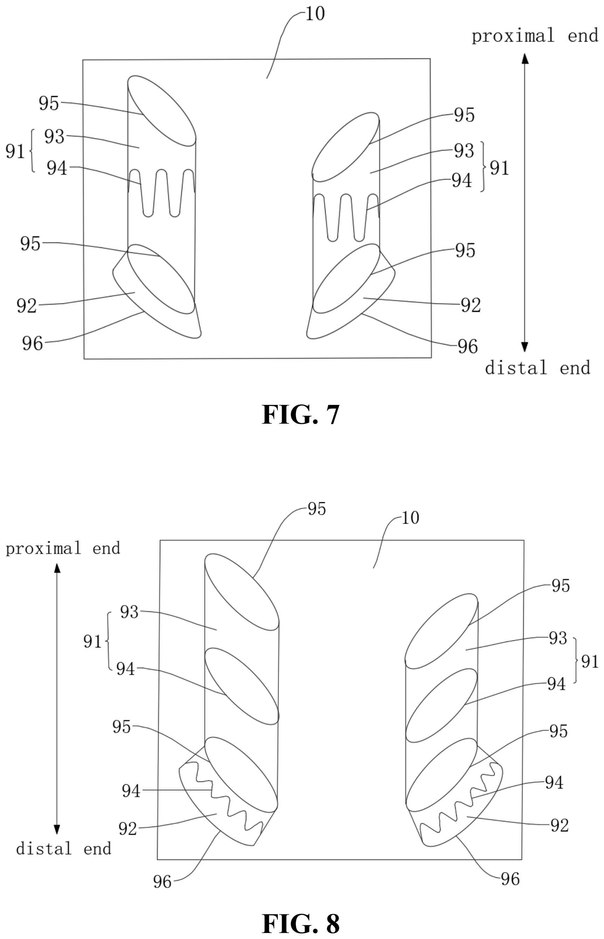

Referring to FIG. 6 and FIG. 7, FIG. 7 is a partial schematic structural view of the fenestrated stent graft of the first embodiment, where the support frame 20 and part of the tubular covering membrane 10 are omitted. The embedded branch tube 90 has two opposite ends in an axial direction of the embedded branch tube 90. One end of the embedded branch tube 90 is fixed to the branch fenestration 12, and the other end of the embedded branch tube 90 extends toward the proximal end of the tubular covering membrane 10, such that the embedded branch tube 90 extends almost entirely along the first tube segment 13.

The diameter of each of the second tube segment 14 and the third tube segment 15 is less than the diameter of the first tube segment 13, so as to reduce the compression on the branch stent graft 140 fitted with the embedded branch tube 90 caused by the tubular covering membrane 10 and the support frame 20 thereon, which is beneficial for improving the deployment success rate of the branch stent graft 140 and ensuring the long-term patency rate of the branch stent graft 140. Compared with the configuration in which the embedded branch tube 90 extends along the second tube segment 14 and/or the third tube segment 15, the configuration in which the embedded branch tube 90 extends almost entirely along the first tube segment 13 is more advantageous in maintaining the blood flow capacity of the tubular covering membrane 10. Furthermore, the configuration in which the embedded branch tube 90 extends almost entirely along the first tube segment 13 provides more deployment space for the branch stent graft 140, enhances its deployment success rate, and helps to promptly restore the blood supply to the branch artery.

In some embodiments, the two embedded branch tubes 90 differ from each other in length in the axial direction of the tubular covering membrane 10, the proximal end of one of the two embedded branch tubes 90 is closer to the proximal end of the tubular covering membrane 10 than the proximal end of the other of the two embedded branch tube 90, such that the proximal ends of the two embedded branch tubes 90 are staggered in the axial direction of the tubular covering membrane 10, which facilitates an increase in the compression ratio of the fenestrated stent graft within the delivery sheath, reduces a radial size of the delivery sheath, thereby lowering the vascular access requirements for endovascular intervention.

The embedded branch tube 90 may be prone to compression and occlusion due to issues such as mural thrombus, intravascular calcified plaques, or irregular vessel cross-sections, making it difficult to ensure the mid- to long-term efficacy of the interventional treatment. In the later stage, it may be necessary to place a bare stent inside the branch stent graft 140 to perform branch artery intervention in order to maintain the patency rate of the branch stent graft 140, which increases the risk of complications, not only failing to achieve the desired therapeutic effect but also causing significant adverse physical and psychological impacts on the patient.

Referring to FIG. 1, FIG. 6, and FIG. 7, the embedded branch tube 90 includes an embedded tube segment 91 and a skirt tube segment 92. The skirt tube segment 92 is sealingly fixed to a distal end of the embedded tube segment 91. The skirt tube segment 92 is of a tubular membrane structure. A distal end of the embedded branch tube 90 is sealingly fixed to the branch fenestration 12 through a distal end of the skirt tube segment 92, such that a distal port of the embedded tube segment 91 is located within the communication cavity 11 of the tubular covering membrane 10. That is, the distal port of the embedded tube segment 91 and the skirt tube segment 92 are recessed relative to the branch fenestration 12, facilitating movement of the branch stent graft 140.

The additional provision of the skirt tube segment 92 is beneficial for increasing a movement space of the branch stent graft 140, i.e., the distal port of the embedded tube segment 91 may move in the axial direction, the circumferential direction, and/or other directions of the tubular covering membrane 10, thereby increasing the movement space of the branch stent graft 140, which helps to further reduce the likelihood of the branch stent graft 140 being compressed and improves the long-term patency of the branch artery. Meanwhile, the additional provision of the skirt tube segment 92 serves a guiding and supporting function, enabling the branch stent graft 140 to better align with the branch fenestration 12, facilitating the insertion of the branch stent graft 140 into the embedded branch tube 90, and improving the deployment accuracy of the branch stent graft 140.

The embedded tube segment 91 includes an embedded membrane 93 and at least one embedded frame 94 fixed to the embedded membrane 93.

In some embodiments, the embedded membrane 93 is of a tubular structure, and one fixation ring 95 is provided and fixed to a proximal port of the embedded membrane 93 and another fixation ring 95 is provided and fixed to a distal port of the embedded membrane 93. A central axis of the fixation ring 95 coincides with a central axis of the embedded membrane 93.

The embedded frame 94 is of a wave shape structure similar to that of the support frame 20. The configuration of the embedded frame 94 is beneficial for improving the anchoring performance of the embedded tube segment 91, thereby enabling the branch stent graft 140 to be more stably inserted into the embedded branch tube 90.

In some embodiments, an axial distance from a distal end of the embedded frame 94 at a distal end of the embedded membrane 93 to a proximal end of the embedded tube segment 91 ranges from 8 mm to 18 mm, such that there is no embedded frame 94 between the distal end of the embedded membrane 93 and a proximal end of the skirt tube segment 92. That is, a region of the embedded membrane 93 between the distal end of the embedded membrane 93 and the proximal end of the skirt tube segment 92 is more flexible, which can provide sufficient movement space for the distal port of the embedded tube segment 91, facilitating a reduction in the risk of compression-induced occlusion after the branch stent graft 140 is inserted into the embedded branch tube 90.

In other embodiments, referring to FIG. 8, which is a partial schematic structural view of the fenestrated stent graft of the first embodiment according to another possible implementation, where the support frame 20 and part of the tubular covering membrane 10 are omitted. The included angle 200A between each two adjacent support rods 201 of the embedded frame 94 may be 180° or 0°, i.e., the embedded frame 94 is configured as an annular structure without a crest 202 and a trough 203, and may not be in a wavy shape. This configuration facilitates a reduction in the difficulty of radially compressing the fenestrated stent graft for containment in a delivery sheath, and may reduce an outer diameter of the delivery sheath to some extent.

In some embodiments, a proximal port of the embedded tube segment 91 is arranged obliquely, i.e., the fixation ring 95 at the proximal port of the embedded tube segment 91 is arranged obliquely. Taking the distal end of the embedded tube segment 91 as a bottom point, an oblique direction of the proximal port of the embedded tube segment 91 may be such that a side of the proximal port closer to a central axis of the embedded tube segment 91 settles toward the distal end of the embedded tube segment 91 relative to a side of the proximal port farther away from the central axis of the embedded tube segment 91.

In other words, the side of the proximal port of the embedded tube segment 91 closer to the central axis of the embedded tube segment 91 is closer to the distal end of the tubular covering membrane 10 than the side of the proximal port of the embedded tube segment 91 farther away from the central axis of the embedded tube segment 91. The distal port of the embedded tube segment 91 is obliquely arranged, i.e., the fixation ring 95 at the distal port of the embedded tube segment 91 is obliquely arranged. The distal port of the embedded tube segment 91 has the same oblique direction as the proximal port of the embedded tube segment 91.

It may be understood that the same oblique direction does not merely mean that a plane where the distal port of the embedded tube segment 91 is located is parallel to a plane where the proximal port of the embedded tube segment 91 is located. An included angle between the plane where the distal port of the embedded tube segment 91 is located and the central axis of the embedded tube segment 91 may or may not be the same as an included angle between the plane where the proximal port of the embedded tube segment 91 is located and the central axis of the embedded tube segment 91.

The fixation ring 95 at the distal port of the embedded tube segment 91 may be bent toward the proximal port of the embedded tube segment 91, such that the plane where the fixation ring 95 is located is an arched curved surface. This configuration enables the fixation ring 95 at the distal port of the embedded tube segment 91 to have good resilience, which helps reduce the compression of the branch stent graft 140 by the fixation ring 95, thereby further improving the long-term patency of the branch artery. A proximal edge of the distal port of the embedded tube segment 91 may be flush with a distal edge of the second fenestration 402, thereby further increasing an axial length of the embedded branch tube 90, which is beneficial for reducing the risk of endoleak after the branch stent graft 140 is fitted with the embedded branch tube 90.

A distal port of the skirt tube segment 92 is obliquely arranged. The distal port of the skirt tube segment 92 may have the same oblique direction as at least one of the distal port of the embedded tube segment 91 or the proximal port of the embedded tube segment 91. It may be understood that the same oblique direction may mean that a plane where the distal port of the skirt tube segment 92 is located is parallel to the plane where the distal port of the embedded tube segment 91 is located, or that the plane where the distal port of the skirt tube segment 92 is located has a tendency to intersect with the plane where the distal port of the embedded tube segment 91 is located. In other words, an included angle between the plane where the distal port of the skirt tube segment 92 is located and the central axis of the embedded tube segment 91 may or may not be the same as an included angle between the plane where the distal port of the embedded tube segment 91 is located and the central axis of the embedded tube segment 91.

In other embodiments, for example, referring to FIG. 8 again, at least one embedded frame 94 may be disposed at the skirt tube segment 92, which will not be repeated herein.

In some embodiments, the distal port of the skirt tube segment 92 is fixed to the branch fenestration 12, and is located on a distal side of the clearance region 100. The distal port of the skirt tube segment 92 is located between the second frame 23 and the third frame 24.

In some embodiments, the distal port of the skirt tube segment 92 is sutured and fixed to the branch fenestration 12 through a suture. A suture loop formed by the suture may serve as a reinforcement ring 96 that reinforces the shape of the branch fenestration 12. The suture loop surrounds at least part of at least two adjacent support rods 201 of the third frame 24, such that the reinforcement ring 96 at least partially cooperates with the at least two adjacent support rods 201 of the third frame 24 to form a ring-shaped structure.

In some embodiments, a circumferential length of a distal end of the branch fenestration 12 is less than a circumferential length of a proximal end of the branch fenestration 12. The distal end of the branch fenestration 12 has a tendency to narrow compared to the proximal end of the branch fenestration 12. This configuration allows the distal port of the embedded tube segment 91 to move more stably within the branch fenestration 12 and is also beneficial for enhancing the wall apposition and structural stability of the branch fenestration 12.

Since the reinforcement ring 96 at least partially cooperates with the at least two adjacent support rods 201 of the third frame 24 to form a ring-shaped structure, the branch fenestration 12 extends along the second tube segment 14 and the third tube segment 15. The second tube segment 14 is in a substantially tapered shape, which allows the branch stent graft to enter the embedded branch tube 90 more precisely through the branch fenestration 12, thereby helping to shorten an operator's learning curve and also reducing the risk of compression of the branch stent graft 140. Meanwhile, as the branch fenestration 12 extends along the second tube segment 14 and the third tube segment 15 and the second tube segment 14 is in a substantially tapered shape, a movement space of the skirt tube segment 92 can be increased, thereby facilitating the avoidance of compression of the branch stent graft 140.

According to exemplary embodiments of the disclosure, in some exemplary embodiments, the reinforcement ring 96 for reinforcing the shape of the branch fenestration 12 may additionally be fixed at the branch fenestration 12. The reinforcement ring 96 is fixed to the branch fenestration 12 through a suture to maintain the shape of the branch fenestration 12. It may be understood that the reinforcement ring 96 at least partially cooperates with the at least two adjacent support rods 201 of the third frame 24 to form a ring-shaped structure.

In some exemplary embodiments, the embedded tube segment 91 is fixed through partial suturing to the inner circumferential wall of the tubular covering membrane 10, i.e., the distal end of the embedded tube segment 91 is fixed to the tubular covering membrane 10 through the skirt tube segment 92, and the proximal end of the embedded tube segment 91 is also fixed to the inner circumferential wall of the tubular covering membrane 10.

In other words, both ends of the embedded tube segment 91 are fixed to the tubular covering membrane 10, while a middle region of the embedded tube segment 91 has no fixation point. This mode of fixation further increases the movement space of the distal port of the embedded tube segment 91, thereby reducing the risk of compression of the branch stent graft 140. Meanwhile, the increased movement space of the distal port of the embedded tube segment 91 is beneficial for adapting to more diverse distribution patterns of branch arteries, and thereby accommodating more diverse lesion vessels.

According to exemplary embodiments of the disclosure, in some exemplary embodiments, the first support ring 403, the second support ring 404, the fixation ring 95, and the reinforcement ring 96 are each provided with a radiopaque marker (not illustrated), which respectively indicate positions of the first support ring 403, the second support ring 404, the fixation ring 95, and the reinforcement ring 96.

The radiopaque marker may be made of materials with good X-ray impermeability, strong corrosion resistance, and excellent biocompatibility, and may include gold, platinum, tantalum, osmium, rhenium, tungsten, iridium, rhodium, or alloys thereof.

The radiopaque marker may also be understood as that the first support ring 403, the second support ring 404, the fixation ring 95, and the reinforcement ring 96 each are made of a radiopaque material. Alternatively, the radiopaque marker may also be understood as that, a radiopaque marker may be disposed on each of the first support ring 403, the second support ring 404, the fixation ring 95, and the reinforcement ring 96, the radiopaque marker on the first support ring 403 at least partially surrounds the first support ring 403, the radiopaque marker on the second support ring 404 at least partially surrounds the second support ring 404, the radiopaque marker on the fixation ring 95 at least partially surrounds the fixation ring 95, and the radiopaque marker on the reinforcement ring 96 at least partially surrounds the reinforcement ring 96.

In a possible application scenario of the fenestrated stent graft, as illustrated in FIG. 9, which is a schematic view illustrating an application scenario of the fenestrated stent graft of the first embodiment for reconstruction of an abdominal aorta 120. The vessel is the abdominal aorta 120. An example is taken in which the fenestrated stent graft is deployed into the abdominal aorta 120, for a juxtarenal abdominal aortic aneurysm formed around a renal region of the abdominal aorta 120, the first tube segment 13 of the fenestrated stent graft may be deployed into a relatively healthy renal vessel of the abdominal aorta 120 to enhance the anchoring performance of the fenestrated stent graft within the target vessel. This allows enhanced wall apposition between the proximal end of the fenestrated stent graft and the vessel wall of the target vessel, which is beneficial for reducing the risk of type I endoleak.

The second tube segment 14 and the third tube segment 15 of the fenestrated stent graft are relatively close to the aneurysm sac 130 of the abdominal aorta 120, thereby providing more space for access of the branch stent graft 140 and reducing the risk of compression of the branch stent graft 140. The branch stent graft 140 is deployed into the fenestration and/or at least one embedded branch tube 90 for reconstruction of at least one branch artery on the abdominal aorta 120 (e.g., the bilateral renal arteries 160 and the superior mesenteric artery 170, etc.).

As illustrated in FIG. 9, multiple branch stent grafts 140 are respectively deployed into the second fenestration 402 and two embedded branch tubes 90 for reconstruction of the bilateral renal arteries 160 and the superior mesenteric artery 170 on the abdominal aorta 120.

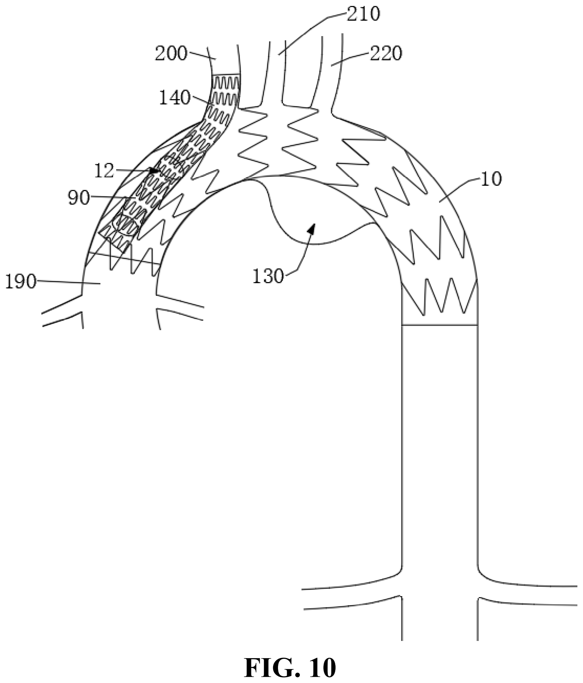

In another possible application scenario of the fenestrated stent graft, as illustrated in FIG. 10, which is a schematic view illustrating a scenario of the fenestrated stent graft of the first embodiment for reconstruction of the aortic arch 190. The blood vessel is the aortic arch 190. An example is taken in which the fenestrated stent graft is deployed into the aortic arch 190 for reconstruction of the brachiocephalic artery 200, for an aneurysm sac 130 or dissection formed in the aortic arch 190, the first tube segment 13 of the fenestrated stent graft may be deployed into the relatively healthy vessel of the aortic arch 190 to enhance anchoring performance of the fenestrated stent graft in the target vessel. This allows enhanced wall apposition between the proximal end of the fenestrated stent graft and the vessel wall of the target vessel, which is beneficial for reducing the risk of type I endoleak. The second tube segment 14 and the third tube segment 15 of the fenestrated stent graft are relatively close to the aneurysm sac 130 of the aortic arch 190, thereby providing more space for access of the branch stent graft 140 and reducing the risk of compression of the branch stent graft 140. The branch stent graft 140 is deployed into the second fenestration 402 and/or at least one embedded branch tube 90 for reconstruction of at least one branch artery on the aortic arch 190 (e.g., the brachiocephalic artery 200, a left common carotid artery 210, and a left subclavian artery 220, etc.).

FIG. 10 illustrates that the branch stent graft 140 is deployed into the second fenestration 402 for reconstruction of the brachiocephalic artery 200 on the aortic arch 190.

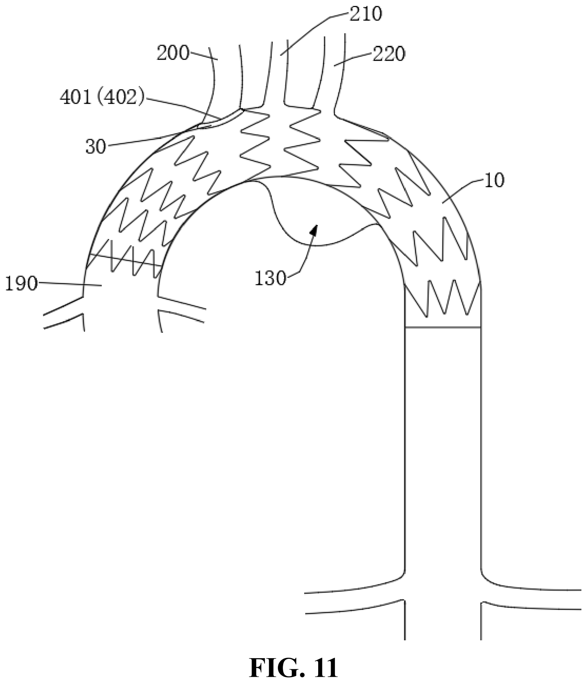

In other exemplary application scenarios, such as illustrated in FIG. 11, FIG. 11 is another schematic view illustrating a scenario of the fenestrated stent graft of the first embodiment for reconstruction of the aortic arch 190, where the first fenestration 401 is aligned with an ostium of least one branch artery on the aortic arch 190.

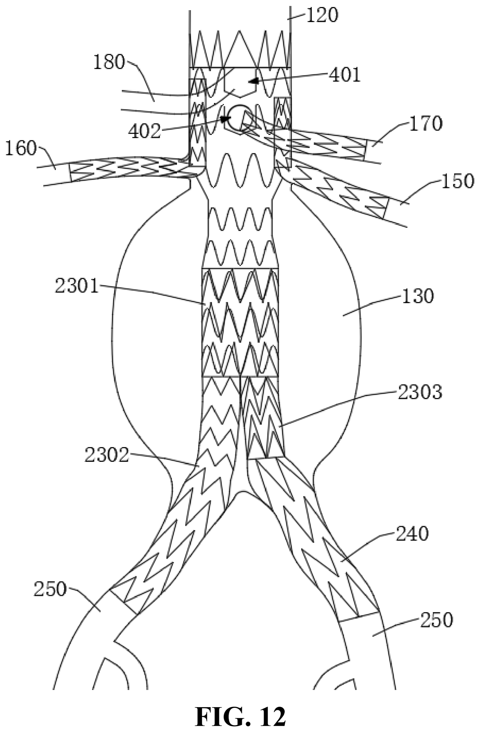

According to a second embodiment of the disclosure, a stent graft system is provided. As illustrated in FIG. 12, the second embodiment differs from at least one implementation of the first embodiment in that the second embodiment further includes a bifurcated stent graft 230. A proximal end of the bifurcated stent graft 230 is fitted with a distal end of the fenestrated stent graft. In other words, the proximal end of the bifurcated stent graft 230 is fitted with at least part of the third tube segment 15 of the fenestrated stent graft. The bifurcated stent graft 230 includes a proximal tube segment 2301, a first side branch 2302, and a second side branch 2303. The first side branch 2302 is fixed to and in communication with the distal end of the proximal tube segment 2301. The second side branch 2303 is also fixed to and in communication with the distal end of the proximal tube segment 2301, such that the proximal tube segment 2301 can shunt along the first side branch 2302 and the second side branch 2303. An axial length of the first side branch 2302 is greater than that of the second side branch 2303.

In some other embodiments, the second embodiment further includes at least one graft extension 240. A proximal end of the graft extension 240 is fitted with a distal end of at least one of the first side branch 2302 or the second side branch 2303.

In this embodiment, the proximal end of the graft extension 240 is fitted with the distal end of the second side branch 2303.

In a possible application scenario of the stent graft system, as illustrated in FIG. 12, which is a schematic view illustrating an application scenario of the stent graft system of the second embodiment for reconstruction of the abdominal aorta 120. The blood vessel is the abdominal aorta 120. An example is taken in which the stent graft system is deployed into the abdominal aorta 120, for a juxtarenal abdominal aortic aneurysm formed around a renal region of the abdominal aorta 120, the first tube segment 13 of the stent graft system may be deployed into a relatively healthy renal vessel of the abdominal aorta 120 to enhance the anchoring performance of the stent graft system within the target vessel. This allows enhanced wall apposition between the proximal end of the stent graft system and the vessel wall of the target vessel, which is beneficial for reducing the risk of type I endoleak.

The second tube segment 14 and the third tube segment 15 of the stent graft system are relatively close to the aneurysm sac 130 of the abdominal aorta 120, thereby providing more space for the access of the branch stent graft 140 and reducing the risk of compression of the branch stent graft 140. The branch stent graft 140 is deployed into the fenestration and/or at least one embedded branch tube 90 for reconstruction of at least one branch artery on the abdominal aorta 120 (e.g., the bilateral renal arteries 160 and the superior mesenteric artery 170, etc.).

As illustrated in FIG. 12, multiple branch stent grafts 140 are respectively deployed into the second fenestration 402 and two embedded branch tubes 90 for reconstruction of the bilateral renal arteries 160 and the superior mesenteric artery 170 on the abdominal aorta 120. The first side branch 2302 and the second side branch 2303 are respectively deployed into two iliac arteries 250, and the graft extension 240 is deployed together with the second side branch 2303 into one of the iliac arteries 250.

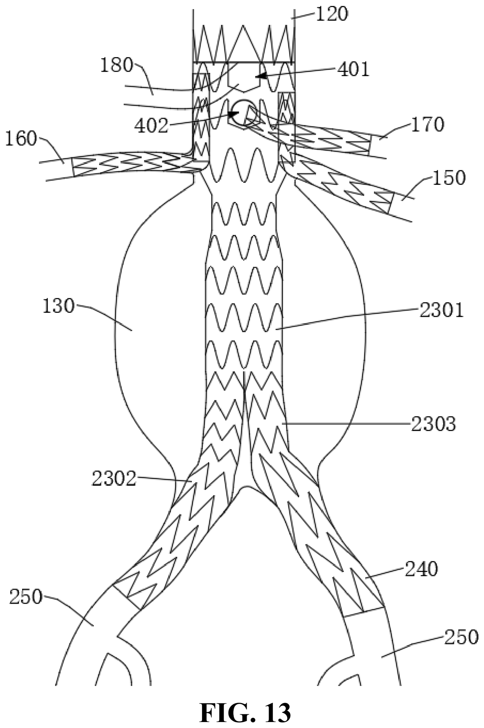

According to a third embodiment of the disclosure, a stent graft system is provided. As illustrated in FIG. 13, the third embodiment differs from at least one implementation of the first embodiment or second embodiment in that the third embodiment further includes a bifurcated stent graft 230, where a proximal end of the bifurcated stent graft 230 is fixed to the distal end of the fenestrated stent graft, and the bifurcated stent graft 230 is integrally formed with the fenestrated stent graft. The bifurcated stent graft 230 includes a proximal tube segment 2301, a first side branch 2302, and a second side branch 2303. The first side branch 2302 is fixed to the distal end of the proximal tube segment 2301 and in communication with the proximal tube segment 2301, and the second side branch 2303 is fixed to the distal end of the proximal tube segment 2301 and in communication with the proximal tube segment 2301, such that the proximal tube segment 2301 can shunt along the first side branch 2302 and the second side branch 2303. The axial length of the first side branch 2302 is greater than that of the second side branch 2303.

In some other embodiments, the third embodiment further includes at least one graft extension 240. The proximal end of the graft extension 240 is fixed to the distal end of at least one of the first side branch 2302 or the second side branch 2303, and the graft extension 240 is integrally formed with at least one of the first side branch 2302 or the second side branch 2303.

In this embodiment, the graft extension 240 is integrally formed with the second side branch 2303.

In a possible application scenario of the stent graft system, as illustrated in FIG. 13, which is a schematic structural view illustrating an application scenario of the stent graft system of the third embodiment for reconstruction of the abdominal aorta 120. The blood vessel is the abdominal aorta 120. An example is taken in which the stent graft system is deployed into the abdominal aorta 120, for a juxtarenal abdominal aortic aneurysm formed around a renal region of the abdominal aorta 120, the first tube segment 13 of the stent graft system may be deployed into a relatively healthy renal vessel of the abdominal aorta 120 to enhance the anchoring performance of the stent graft system within the target vessel. This allows enhanced wall apposition between the proximal end of the stent graft system and the vessel wall of the target vessel, which is beneficial for reducing the risk of type I endoleak.

The second tube segment 14 and the third tube segment 15 of the stent graft system are relatively close to the aneurysm sac 130 of the abdominal aorta 120, thereby providing more space for the access of the branch stent graft 140 and reducing the risk of compression of the branch stent graft 140. The branch stent graft 140 is deployed into the fenestration and/or at least one embedded branch tube 90 for reconstruction of at least one branch artery on the abdominal aorta 120 (e.g., the bilateral renal arteries 160 and the superior mesenteric artery 170, etc.).

As illustrated in FIG. 13, multiple branch stent grafts 140 are respectively deployed into the second fenestration 402 and two embedded branch tubes 90 for reconstruction of the bilateral renal arteries 160 and the superior mesenteric artery 170 on the abdominal aorta 120. The first side branch 2302 and the second side branch 2303 are deployed into two iliac arteries 250, respectively, and the graft extension 240 is deployed together with the second side branch 2303 into one of the iliac arteries 250.

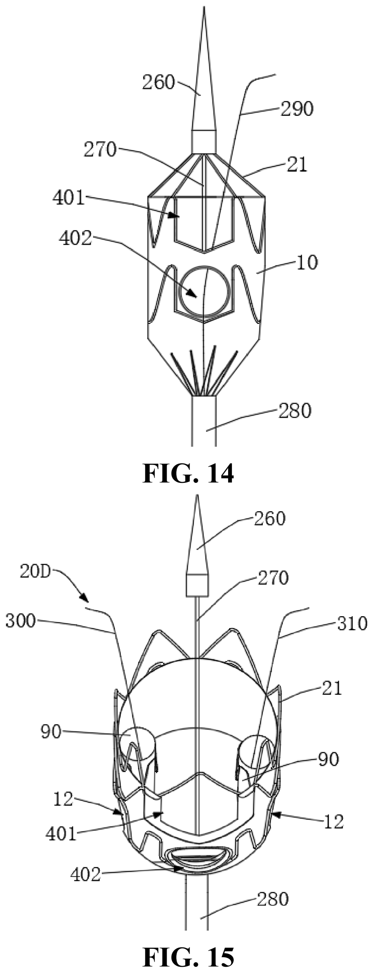

According to a fourth embodiment of the disclosure, a stent graft system is provided. The fourth embodiment differs from at least one implementation of the first embodiment, the second embodiment, or the third embodiment in that the fourth embodiment further includes an interventional device for delivering the fenestrated stent graft to a target position (for example, a target vessel). Referring to FIG. 14 and FIG. 15, FIG. 14 is a schematic structural view of the fenestrated stent graft, that is at least partially self-expanded, of the stent graft system of the fourth embodiment. Specifically, the delivery sheath 280 is moved axially a certain distance toward a distal end of the delivery inner shaft 270, such that part of the fenestrated stent graft close to the proximal end is exposed relative to the delivery sheath 280 and self-expands. FIG. 15 is a schematic structural view of the fenestrated stent graft, that is at least partially self-expanded, of the stent graft system of the fourth embodiment, viewed from another angle. Specifically, the delivery sheath 280 is moved axially a certain distance toward the distal end of the delivery inner shaft 270, such that part of the fenestrated stent graft close to the proximal end is exposed relative to the delivery sheath 280 and self-expands.

The interventional device includes a tip 260, a delivery inner shaft 270, a delivery sheath 280, a delivery handle (not illustrated), and at least three pre-embedded guidewires 20D. The tip 260 is fixed to a proximal end of the delivery inner shaft 270. The delivery sheath 280 is sleeved over the delivery inner shaft 270. A radial dimension of an inner cavity of the delivery sheath 280 is less than a radial dimension of a distal end of the tip 260, such that a proximal end of the delivery sheath 280 can contact the distal end of the tip 260. This is beneficial for preventing the delivery sheath 280 from extending beyond the proximal end of the delivery inner shaft 270 when the delivery sheath 280 moves toward a proximal end of the interventional device. A gap is defined between the delivery sheath 280 and the delivery inner shaft 270 for accommodating the radially compressed fenestrated stent graft.

It may be noted that the radially compressed fenestrated stent graft refers to a stent graft having a radial dimension less than that after self-expansion.

The delivery inner shaft 270 is configured to detachably fix the fenestrated stent graft. For example, the fenestrated stent graft is hooked to the delivery inner shaft 270 through the crest 202 of the bare stent 21. An axial length of the fenestrated stent graft is less than an axial length of the delivery inner shaft 270, such that the fenestrated stent graft is located at part of the delivery inner shaft 270 close to the proximal end. The delivery handle is configured to operate the delivery sheath 280 to move in an axial direction of the delivery inner shaft 270. When the delivery handle controls the delivery sheath 280 to move axially toward the distal end of the delivery inner shaft 270, the fenestrated stent graft is exposed relative to the delivery sheath 280. When the delivery handle controls the delivery sheath 280 to move axially toward the proximal end of the delivery inner shaft 270, and the proximal end of the delivery sheath 280 does not exceed the proximal end of the delivery inner shaft 270, the delivery sheath 280 encloses the fenestrated stent graft such that the fenestrated stent graft is confined within the gap between the delivery sheath 280 and the delivery inner shaft 270.

For ease of distinction, the three pre-embedded guidewires 20D are respectively designated as a first pre-embedded guidewire 290, a second pre-embedded guidewire 300, and a third pre-embedded guidewire 310. The first pre-embedded guidewire 290 is at least partially located outside the interventional device, at least partially extends through the delivery sheath 280, and at least partially extends out of the proximal end of the fenestrated stent graft. For part of the first pre-embedded guidewire 290 that is located within the delivery sheath 280, part of the portion of the first pre-embedded guidewire 290 that is on a distal side of the second fenestration 402 is located outside the fenestrated stent graft, and part of the portion of the first pre-embedded guidewire 290 that is adjacent to the proximal end of the fenestrated stent graft passes through the second fenestration 402 and is located within the communication cavity 11 of the fenestrated stent graft. A proximal end of the first pre-embedded guidewire 290 extends beyond the proximal end of the fenestrated stent graft and the proximal end of the delivery sheath 280.

For example, in some exemplary embodiments, the first pre-embedded guidewire 290 extends through the delivery sheath 280. The proximal end of the first pre-embedded guidewire 290 enters the second fenestration 402 from the outside of the fenestrated stent graft and then enters the communication cavity 11 of the fenestrated stent graft, and continues moving toward the proximal end of the delivery inner shaft 270 and the proximal end of the fenestrated stent graft until the proximal end of the first pre-embedded guidewire 290 extends beyond the proximal end of the fenestrated stent graft and the proximal end of the delivery sheath 280.

The second pre-embedded guidewire 300 is at least partially located outside the interventional device, and at least partially extends through one of the embedded branch tubes 90. The second pre-embedded guidewire 300 at least partially extends out of the proximal end of the fenestrated stent graft. The third pre-embedded guidewire 310 is at least partially located outside the interventional device, and at least partially extends through another embedded branch tube 90. The third pre-embedded guidewire 310 at least partially extends out of the proximal end of the fenestrated stent graft.

In some exemplary embodiments, the second pre-embedded guidewire 300 is at least partially located outside the interventional device and at least partially extends through one of the embedded branch tubes 90, and the proximal end of the second pre-embedded guidewire 300 is located within the communication cavity 11 of the fenestrated stent graft. The third pre-embedded guidewire 310 is at least partially located outside the interventional device and at least partially extends through another embedded branch tube 90, and the proximal end of the third pre-embedded guidewire 310 is located within the communication cavity 11 of the fenestrated stent graft.

In a fifth embodiment of the disclosure, a method for suturing a fenestrated stent graft is provided, where the enveloping band 30 is fixed to the fenestrated stent graft (for example, the tubular covering membrane 10 and/or the support frame 20) through a suture.

In some other embodiments, the enveloping band 30 is fixed to the fenestrated stent graft by means such as adhering, stamping, attaching, embedding, or thermal pressing.

In this embodiment, an example is taken in which the enveloping band 30 is fixed to the fenestrated stent graft through suturing, which does not limit the method for fixing the enveloping band 30.

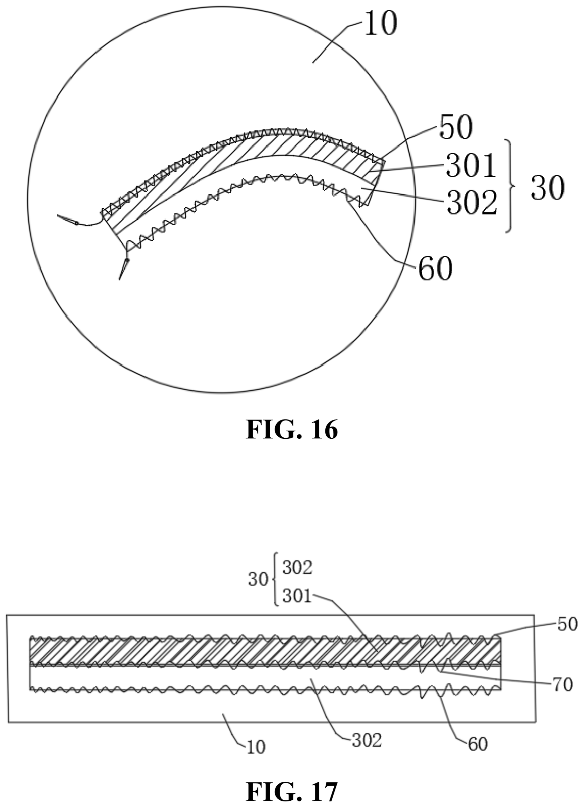

Referring to FIG. 16, which is a partial schematic view of FIG. 3. In some other embodiments, the first region 301 is formed by folding two-layers of membrane structure, and the second region 302 is formed by a single-layer of membrane structure. An exemplary method for suturing the enveloping band 30 may include the following.

At S 10, the enveloping band 30 is disposed to surround the fenestration 40. The enveloping band 30 has a first side edge and a second side edge. The first side edge is closer to the fenestration 40 than the second side edge. The first side edge is folded to form the first region 301 that has two layers of membrane structure, and the second side edge forms the second region 302 that has a single-layer of membrane structure.

At S20, at least part of the first region 301 covers and surrounds at least part of the inner edge 405 of the fenestration 40, and at least part of the first region 301 covers and surrounds at least part of the outer edge 406 of the fenestration 40; the first region 301 is fixed to the fenestrated stent graft through a suture, and the second region 302 is fixed to the fenestrated stent graft through a suture.

In some other embodiments, S20 may also be carried out as follows. At least part of the first region 301 covers and surrounds at least part of the inner edge 405 of the fenestration 40, and at least part of the first region 301 covers and surrounds at least part of the outer edge 406 of the fenestration 40. The first region 301 is fixed to the fenestrated stent graft through suturing with one or more of the following suture techniques such as running stitch, basting stitch, setting stitch, whip stitch, overcast stitch, backstitch, overlock stitch, triangular stitch, straight stitch, tack stitch, and embroidery stitch. The second region 302 is fixed to the fenestrated stent graft through suturing with one or more of the following suture techniques such as running stitch, basting stitch, setting stitch, whip stitch, overcast stitch, backstitch, overlock stitch, triangular stitch, straight stitch, tack stitch, and embroidery stitch.

In some other embodiments, as for the operations carried out at S20, the first region 301 and the second region 302 of the enveloping band 30 on the first fenestration 401 may be fixed to the fenestrated stent graft as follows.

At S201, a needle bar connected to a lead end of a suture penetrates from the inner circumferential wall of the tubular covering membrane 10 to the outer circumferential wall of the first region 301, and a knot is formed at a tail end of the suture and fixed to the fenestrated stent graft to form an initial knot point (not illustrated), which is located at the inner circumferential wall of the fenestrated stent graft.

At S202, after the suture penetrates from the outer circumferential wall of the first region 301, the suture is wrapped around the first support ring 403 and enters the first fenestration 401, then penetrates from the inner circumferential wall of the tubular covering membrane 10 to the outer circumferential wall of the first region 301 to form a first wrapping coil. The first wrapping coil wraps around the first support ring 403.

Operations carried out at S202 are repeatedly performed to form a wrapping suture group 50 that wraps around the first support ring 403 and fixes the first region 301 to the fenestrated stent graft. The shape of the wrapping suture group 50 varies according to the shape of the first support ring 403. At this point, the suture has penetrated from the inner circumferential wall of the tubular covering membrane 10 to the outer circumferential wall of the first region 301.

At S203, after the suture forms the wrapping suture group 50 at the first region 301 and penetrates from the inner circumferential wall of the tubular covering membrane 10 to the outer circumferential wall of the first region 301, the suture is not tied. The needle bar pulls the suture to wrap around the first support ring 403, enter the first fenestration 401, and penetrate from the inner circumferential wall of the tubular covering membrane 10 to the outer circumferential wall of the second region 302.

At S204, the needle bar pulls the suture to wrap around an edge of the second region 302 that is away from the first region 301, and then penetrates from the outer circumferential wall of the tubular covering membrane 10 to the inner circumferential wall of the tubular covering membrane 10 to form a second wrapping coil. The second wrapping coil is at least partially located on the outer circumferential wall of the tubular covering membrane 10 and the outer circumferential wall of the second region 302, that is, the second wrapping coil is in contact with both the outer circumferential wall of the second region 302 and the outer circumferential wall of the tubular covering membrane 10, thereby fixing the second region 302 to the fenestrated stent graft through suturing.

Operations carried out at S204 are repeatedly performed to form a fixing suture group 60 that fixes the second region 302 to the fenestrated stent graft. The suture is tied and fixed to the inner circumferential wall of the tubular covering membrane 10 to form a terminal knot point (not illustrated).

The method for fixing the first region 301 and the second region 302 of the enveloping band 30 on the second fenestration 402 may be the same as the method for fixing the enveloping band 30 on the first fenestration 401, which will not be repeated here.

In some other embodiments, referring to FIG. 17, which is a partial schematic view of FIG. 3 according to another possible embodiment. For the enveloping band 30 on the first fenestration 401 and the second fenestration 402, a side of the first region 301 close to the second region 302 may be fixed to the fenestrated stent graft through suturing. For example, the side of the first region 301 close to the second region 302 may be fixed to the fenestrated stent graft through suturing with one or more of the following suture techniques such as running stitch, basting stitch, setting stitch, whip stitch, overcast stitch, backstitch, overlock stitch, triangular stitch, straight stitch, tack stitch, and embroidery stitch. The suturing on the side of the first region 301 close to the second region 302 forms a suture group 70, such that a smooth transition is formed between the first region 301 and the second region 302 of the enveloping band 30, thereby enhancing the wall apposition of the enveloping band 30, and helping reduce the risk of endoleak at the proximal ends of the fenestration 40 and the fenestrated stent graft.

The above are preferred embodiments of the disclosure and are not intended to limit the scope of protection of the disclosure. Therefore, any equivalent variations made based on the structure, shape, and principle of the disclosure shall fall within the scope of protection of the disclosure.

## Claims

1. A fenestrated stent graft, comprising:
a tubular covering membrane, wherein the tubular covering membrane is a tubular structure with openings at two opposite ends, defines a communication cavity, and is provided with at least one fenestration;
a support frame, wherein the support frame is implemented as a plurality of support frames that are arranged in an axial direction of the tubular covering membrane; and
at least one enveloping band surrounding the at least one fenestration, wherein for each of the at least one enveloping band, the enveloping band is at least partially located on an outer circumferential wall of the tubular covering membrane and comprises a first region and a second region, wherein the first region is disposed in close contact with and surrounds a corresponding one of the at least one fenestration, the second region is disposed in close contact with and surrounds a side of the first region away from the corresponding one of the at least one fenestration, and a thickness of the first region is greater than a thickness of the second region.

2. The fenestrated stent graft according to claim 1, wherein for each of the plurality of support frames, the support frame is located on the outer circumferential wall of the tubular covering membrane, and the thickness of the first region is greater than a wire diameter of the support frame.

3. The fenestrated stent graft according to claim 2, wherein the thickness of the second region is less than the wire diameter of the support frame.

4. The fenestrated stent graft according to claim 1, wherein a wire diameter of the support frame ranges from 0.30 mm to 0.50 mm, the thickness of the first region of the enveloping band ranges from 0.35 mm to 0.60 mm, and the thickness of the second region of the enveloping band ranges from 0.15 mm to 0.28 mm.

5. The fenestrated stent graft according to claim 1, wherein the enveloping band is made of biocompatible fabric, the tubular covering membrane is made of biocompatible fabric, and a fabric density of the biocompatible fabric of the enveloping band is less than a fabric density of the biocompatible fabric of the tubular covering membrane, such that the enveloping band is fluffier and softer than the tubular covering membrane.

6. The fenestrated stent graft according to claim 1, wherein
for each of the at least one fenestration, the fenestration comprises an inner edge and an outer edge, wherein the first region covers and surrounds the inner edge and at least part of the outer edge of the fenestration, and the second region covers and surrounds at least part of the outer edge of the fenestration; or
for each of the at least one fenestration, the fenestration comprises an inner edge and an outer edge, wherein the first region covers and surrounds at least part of the outer edge of the fenestration, and the second region covers and surrounds at least part of the outer edge of the fenestration.

7. The fenestrated stent graft according to claim 1, wherein a side of the first region adjacent to the second region is fixed to the fenestrated stent graft through a suture.

8. The fenestrated stent graft according to any one of claims 1 to 7, wherein
the at least one fenestration comprises at least one first fenestration and at least one second fenestration, wherein the at least one first fenestration and the at least one second fenestration are arranged in the axial direction of the tubular covering membrane, and the at least one first fenestration is closer to a proximal end of the tubular covering membrane than the at least one second fenestration; and
the at least one enveloping band is in close contact with and surrounds the at least one first fenestration, and the at least one enveloping band is disposed in close contact with and surrounds the at least one second fenestration.

9. The fenestrated stent graft according to claim 8, wherein
for each of the at least one first fenestration, the first fenestration penetrates through the proximal end of the tubular covering membrane such that the first fenestration forms an open-loop structure, and for each of the at least one second fenestration, the second fenestration is a closed-loop structure; and
the fenestrated stent graft comprises a first support ring and a second support ring both fixed to the tubular covering membrane, the first support ring surrounds the first fenestration, and the second support ring surrounds the second fenestration.

10. The fenestrated stent graft according to claim 8, wherein for each of the at least one second fenestration, an extension tube segment is fixed within the second fenestration, wherein the extension tube segment is located within the communication cavity of the tubular covering membrane, the second fenestration is in communication with the communication cavity of the tubular covering membrane through the extension tube segment, the extension tube segment comprises an extension covering membrane in a tubular shape and at least one wavy frame fixed to the extension covering membrane, and an axial length of the extension tube segment ranges from 2.5 mm to 3.5 mm.

11. The fenestrated stent graft according to any one of claims 1 to 7, wherein
the support frame comprises a plurality of support rods connected sequentially at angles, each two adjacent angles among angles defined by the plurality of support rods in a circumferential direction of the tubular covering membrane define a crest and a trough, and the crest is closer to the proximal end of the tubular covering membrane than the trough;
in the axial direction of the tubular covering membrane, at least one first reinforced anti-leakage region and at least one second reinforced anti-leakage region are provided on two opposite circumferential sides of a central axis of the at least one fenestration, respectively; and
each of the first reinforced anti-leakage region and the second reinforced anti-leakage region comprises at least one density-enhanced region, wherein each of the at least one density-enhanced region is formed by at least two adjacent support rods of the support frame that are connected to each other, and a maximum circumferential length of each two adjacent support rods in the density-enhanced region is less than a maximum circumferential length of each two other adjacent support rods of the support frame.

12. The fenestrated stent graft according to claim 11, wherein
the at least one fenestration comprises a first fenestration, the support frame comprises a bare stent in an annular shape, the bare stent is coaxial with the tubular covering membrane, troughs of the bare stent are fixed to the tubular covering membrane, and crests of the bare stent and at least part of axial portions of the plurality of support rods of the bare stent extend beyond the proximal end of the tubular covering membrane; and
in the circumferential direction of the tubular covering membrane, two adjacent support rods define an avoidance region across the first fenestration, and distal ends of the two adjacent support rods in the avoidance region are located on two opposite sides of the first fenestration, respectively.

13. The fenestrated stent graft according to claim 12, wherein
in the circumferential direction of the tubular covering membrane, at least one density-enhanced region is formed on each of two opposite sides of the avoidance region and each density-enhanced region is referred to as a bare-end density-enhanced region;
each bare-end density-enhanced region is formed by two adjacent support rods of the bare stent that are connected to each other, a circumferential length between distal ends of the two adjacent support rods in the avoidance region is greater than a circumferential length between distal ends of two adjacent support rods forming each of other crests of the bare stent, and a circumferential length between distal ends of the two adjacent support rods in the bare-end density-enhanced region is less than the circumferential length between the distal ends of the two adjacent support rods forming each of the other crests of the bare stent; and
the two adjacent support rods in the bare-end density-enhanced region are adjacent to the two adjacent support rods in the avoidance region in the circumferential direction of the tubular covering membrane.

14. The fenestrated stent graft according to claim 13, wherein a distance between the distal ends of the two adjacent support rods in the avoidance region accounts for 17% to 24% of a circumferential length of the proximal end of the tubular covering membrane, and a distance between the distal ends of the two adjacent support rods in the density-enhanced region of the bare stent accounts for 5% to 10% of the circumferential length of the proximal end of the tubular covering membrane.

15. The fenestrated stent graft according to claim 11, wherein
the at least one fenestration comprises at least one first fenestration and at least one second fenestration, the at least one first fenestration and the at least one second fenestration are arranged in the axial direction of the tubular covering membrane, and the at least one first fenestration is closer to the proximal end of the tubular covering membrane than the at least one second fenestration; and
the plurality of support frame comprise a first frame, a second frame, a third frame, and a plurality of fourth frames that are coaxially arranged with the tubular covering membrane, the first frame, the second frame, the third frame, and the plurality of fourth frames are arranged in sequence from a proximal end to a distal end in the axial direction of the tubular covering membrane, and the at least one first fenestration is located on a proximal side of the first frame, and the at least one second fenestration is located between the first frame and the second frame.

16. The fenestrated stent graft according to claim 15, wherein
the first reinforced anti-leakage region comprises at least two density-enhanced regions, namely a proximal-end density-enhanced region and a reinforced density-enhanced region that are arranged in the axial direction of the tubular covering membrane;
the second reinforced anti-leakage region comprises at least two density-enhanced regions, namely a proximal-end density-enhanced region and a reinforced density-enhanced region that are arranged in the axial direction of the tubular covering membrane; and
the proximal-end density-enhanced region of the first reinforced anti-leakage region and the proximal-end density-enhanced region of the second reinforced anti-leakage region are located on two opposite circumferential sides of the at least one first fenestration, respectively, and the reinforced density-enhanced region of the first reinforced anti-leakage region and the reinforced density-enhanced region of the second reinforced anti-leakage region are located on two opposite circumferential sides of the at least one second fenestration, respectively.

17. The fenestrated stent graft according to claim 16, wherein
for each of the at least one first fenestration, the first fenestration penetrates through the proximal end of the tubular covering membrane such that the first fenestration forms an open-loop structure, the fenestrated stent graft comprises a first support ring fixed to the tubular covering membrane, and the first support ring surrounds the first fenestration;
the first support ring is formed by two adjacent support rods of the first frame, which are a first support rod and a second support rod, respectively, and a distal end of the first support rod and a distal end of the second support rod are connected each other at an angle to form a trough, which is referred to as a support trough; and
each of the first support rod and the second support rod comprises a first connecting rod and a second connecting rod that are connected to each other in the axial direction, the first connecting rod is closer to the proximal end of the tubular covering membrane than the second connecting rod, and a connecting trough is formed by a distal end of the first connecting rod and a proximal end of the second connecting rod that are connected to each other at an angle.

18. The fenestrated stent graft according to claim 17, wherein
the proximal-end density-enhanced region is formed by two adjacent support rods of the first frame that are connected to each other, and the two adjacent support rods in the proximal-end density-enhanced region are connected to each other at an angle to form one crest of the first frame;
one support rod in the proximal-end density-enhanced region of the first reinforced anti-leakage region serves as the first support rod, and one support rod in the proximal-end density-enhanced region of the second reinforced anti-leakage region serves as the second support rod; and
a circumferential length from a distal end of one support rod in the proximal-end density-enhanced region of the first reinforced anti-leakage region, that is away from the first fenestration, to an extension line of the first connecting rod of the first support rod, and a circumferential length from a distal end of one support rod in the proximal-end density-enhanced region of the second reinforced anti-leakage region, that is away from the first fenestration, to an extension line of the first connecting rod of the second support rod, are both less than a circumferential length between distal ends of two adjacent support rods forming each of other crests of the first frame.

19. The fenestrated stent graft according to claim 16, wherein
two adjacent support rods of the second frame form an attaching ring, wherein the two support rods of the attaching ring are referred to as a third support rod and a fourth support rod, respectively, and a trough formed by a distal end of the third support rod and a distal end of the fourth support rod that are connected to each other at an angle is referred to as an attaching trough;
each of the third support rod and the fourth support rod comprises a third connecting rod and a fourth connecting rod that are connected to each other in the axial direction, the third connecting rod is closer to the proximal end of the tubular covering membrane than the fourth connecting rod, and a connecting trough is formed by a distal end of the third connecting rod and a proximal end of the fourth connecting rod that are connected to each other at an angle;
the at least one fenestration comprises a second support ring fixed to the tubular covering membrane, wherein the second support ring surrounds a corresponding one of the at least one second fenestration; and
the second support ring is located on a proximal side of the attaching ring, such that the attaching ring forms an open-loop structure partially surrounding the second support ring.

20. The fenestrated stent graft according to claim 19, wherein
the reinforced density-enhanced region is formed by two adjacent support rods of the second frame that are connected to each other, the two adjacent support rods in the reinforced density-enhanced region are connected to each other at an angle to form one crest of the second frame;
one support rod in the reinforced density-enhanced region of the first reinforced anti-leakage region serves as the third support rod, and one support rod in the reinforced density-enhanced region of the second reinforced anti-leakage region serves as the fourth support rod; and
a circumferential length from a distal end of one support rod in the reinforced density-enhanced region of the first reinforced anti-leakage region, that is away from the at least one second fenestration, to an extension line of the third connecting rod of the third support rod, and a circumferential length from a distal end of one support rod in the reinforced density-enhanced region of the second reinforced anti-leakage region, that is away from the at least one second fenestration, to an extension line of the third connecting rod of the fourth support rod, are both less than a circumferential length between distal ends of two adjacent support rods forming each of other crests of the first frame.

21. The fenestrated stent graft according to claim 19, wherein for each of the at least one second fenestration, the enveloping band on the second fenestration is located between the attaching ring and the second support ring.

22. The fenestrated stent graft according to claim 19, wherein the second frame has at least one anchoring region, for each of the at least one anchoring region, a distal end of the anchoring region is closer to the proximal end of the tubular covering membrane than other regions of the second frame, and an axial length of the anchoring region is greater than that of each of the other regions of the second frame.

23. The fenestrated stent graft according to claim 22, wherein
in the circumferential direction of the tubular covering membrane, at least two troughs adjacent to the attaching trough comprise a first anchoring trough and a second anchoring trough, all the first anchoring trough, the second anchoring trough, and the attaching trough are farther away from the proximal end of the tubular covering membrane than other troughs of the second frame; and
two adjacent support rods forming the first anchoring trough, two adjacent support rods forming the second anchoring trough, and two adjacent support rods forming the attaching trough each have a longer axial length than other support rods forming other troughs of the second frame, to form the anchoring region.

24. The fenestrated stent graft according to claim 23, wherein in the axial direction of the tubular covering membrane, distances from distal ends of the two adjacent support rods forming the first anchoring trough, distal ends of the two adjacent support rods forming the second anchoring trough, and distal ends of the two support rods forming the attaching trough to distal ends of the support rods forming other troughs of the second frame cooperatively define an axial length of the anchoring region, and the axial length of the anchoring region ranges from 3 mm to 5 mm.

25. The fenestrated stent graft according to claim 15, wherein
the tubular covering membrane comprises a first tube segment, a second tube segment, and a third tube segment that are sequentially connected in the axial direction of the tubular covering membrane from the proximal end of the tubular covering membrane to the distal end of the tubular covering membrane;
the first tube segment is of a constant-diameter structure, the third tube segment is of at least one of a constant-diameter structure or a non-constant-diameter structure, the second tube segment is of a tapered non-constant-diameter structure, and a diameter of the second tube segment gradually decreases in a direction from the proximal end of the tubular covering membrane to the distal end of the tubular covering membrane;
a diameter of the first tube segment connected to a proximal end of the second tube segment is greater than a diameter of the third tube segment connected to a distal end of the second tube segment; and
the first frame and the second frame are located on the first tube segment, the third frame is located on the second tube segment, such that the third frame forms a corresponding tapered structure, and the plurality of fourth frames are located on the third tube segment.

26. The fenestrated stent graft according to claim 25, wherein
the fenestrated stent graft further comprises at least two embedded branch tubes, wherein the at least two embedded branch tubes are configured to be fitted with branch stent grafts for reconstruction of branch arteries, the at least two embedded branch tubes are fixed to an inner circumferential wall of the tubular covering membrane, and are located on two opposite sides of the at least one fenestration in the circumferential direction of the tubular covering membrane, respectively;
the tubular covering membrane further defines at least two branch fenestrations, the at least two embedded branch tubes are fixed to the at least two branch fenestrations, respectively, and the at least two branch fenestrations are in communication with the communication cavity of the tubular covering membrane through the at least two embedded branch tubes; and
the at least two branch fenestrations are located between the second frame and the third frame, such that the at least two branch fenestrations extend from the first tube segment to the second tube segment.

27. The fenestrated stent graft according to claim 26, wherein
at least one clearance region is provided on each of two opposite sides of the at least one second fenestration in the circumferential direction of the tubular covering membrane; and
for each of the at least one clearance region, the clearance region is formed on the second frame, and a distal end of the clearance region is closer to the proximal end of the tubular covering membrane than a distal end of each of other regions of the second frame, and an axial length of the clearance region is less than an axial length of each of other regions of the second frame.

28. The fenestrated stent graft according to claim 27, wherein
the clearance region is formed by two adjacent support rods of the second frame that are connected to one another, and a trough, formed by distal ends of the two support rods forming the clearance region that are connected to one another at an angle, is referred to as a clearance trough;
a length of each of the two adjacent support rods in the clearance region in the axial direction of the tubular covering membrane is less than a length of each of other support rods of the second frame in the axial direction of the tubular covering membrane;
the clearance trough is closer to the proximal end of the tubular covering membrane than each of other troughs of the second frame; and
for each of the at least two branch fenestrations, the branch fenestration is located between the clearance trough and a trough of the third frame adjacent to the clearance trough in the axial direction of the tubular covering membrane.

29. The fenestrated stent graft according to claim 26, wherein
for each of the at least two embedded branch tubes, the embedded branch tube has two ports that are opposite to each other in an axial direction of the embedded branch tube, one of the two ports of the embedded branch tube is fixed to the branch fenestration, and the other of the two ports of the embedded branch tube extends toward the proximal end of the tubular covering membrane, such that the embedded branch tube extends along the first tube segment; and
the at least two embedded branch tubes differ from each other in length in the axial direction of the tubular covering membrane, such that a proximal end of one of the at least two embedded branch tubes is closer to the proximal end of the tubular covering membrane than a proximal end of another one of the at least two embedded branch tubes.

30. The fenestrated stent graft according to claim 26, wherein for each of the at least two embedded branch tubes, the embedded branch tube comprises an embedded tube segment and a skirt tube segment, the skirt tube segment is sealingly fixed to a distal port of the embedded tube segment, and a distal port of the embedded branch tube is sealingly fixed to a corresponding one of the at least two branch fenestrations through a distal port of the skirt tube segment, such that the distal port of the embedded tube segment is located within the communication cavity.

31. The fenestrated stent graft according to claim 30, wherein the embedded tube segment comprises an embedded membrane and at least one embedded frame fixed to the embedded membrane, the embedded membrane is in a tubular shape, at least one of a proximal port of the embedded membrane or a distal port of the embedded membrane is fixed to a fixation ring, and a distance, in the axial direction of the tubular covering membrane, from a distal end of each of the at least one embedded frame to a proximal end of the embedded tube segment ranges from 8 mm to 18 mm.

32. The fenestrated stent graft according to claim 31, wherein both the proximal port of the embedded tube segment and the distal port of the embedded tube segment are arranged obliquely, and the fixation ring at the distal port of the embedded tube segment is bent toward the proximal port of the embedded tube segment, such that a surface where the distal port of the embedded tube segment is located is an arched curved surface.

33. The fenestrated stent graft according to claim 30, wherein
the distal port of the skirt tube segment is fixed with a reinforcement ring, and the distal port of the skirt tube segment is arranged obliquely;
the reinforcement ring at least partially cooperates with at least two adjacent support rods of the third frame that form the trough to form a closed-loop structure; and
for each of the at least two branch fenestrations, a circumferential length of a distal end of the branch fenestration is less than a circumferential length of a proximal end of the branch fenestration.

34. The fenestrated stent graft according to claim 30, wherein in an axial direction of the embedded tube segment, both ends of the embedded tube segment are fixed to the inner circumferential wall of the tubular covering membrane, and a middle region of the embedded tube segment has no fixation point.

35. The fenestrated stent graft according to claim 30, wherein a proximal edge of the distal port of the embedded tube segment is flush with a distal edge of a corresponding one of the at least one second fenestration.

36. A stent graft system, comprising the fenestrated stent graft according to any one of claims 1 to 35, a bifurcated stent graft, and at least one graft extension, wherein
a proximal end of the bifurcated stent graft is fitted with a distal end of the fenestrated stent graft; and
the bifurcated stent graft comprises a proximal tube segment, a first side branch, and a second side branch, the first side branch is fixed to a distal end of the proximal tube segment and in communication with the proximal tube segment, and the second side branch is fixed to the distal end of the proximal tube segment and in communication with the proximal tube segment, such that the proximal tube segment is configured to shunt along the first side branch and the second side branch, and a proximal end of the at least one graft extension is fitted with a distal end of at least one of the first side branch or the second side branch.

37. A stent graft system, comprising the fenestrated stent graft according to any one of claims 1 to 35, a bifurcated stent graft, and at least one graft extension, wherein
a proximal end of the bifurcated stent graft is fixed to a distal end of the fenestrated stent graft, and the bifurcated stent graft is integrally formed with the fenestrated stent graft;
the bifurcated stent graft comprises a proximal tube segment, a first side branch, and a second side branch, the first side branch is fixed to a distal end of the proximal tube segment and in communication with the proximal tube segment, and the second side branch is fixed to the distal end of the proximal tube segment and in communication with the proximal tube segment, such that the proximal tube segment is configured to shunt along the first side branch and the second side branch; and
a proximal end of the at least one graft extension is fixed to a distal end of at least one of the first side branch or the second side branch, and the at least one graft extension is integrally formed with at least one of the first side branch or the second side branch.

38. A stent graft system, comprising the fenestrated stent graft according to any one of claims 1 to 35, and an interventional device for delivering the fenestrated stent graft to a target position, wherein
the interventional device comprises a tip, a delivery inner shaft fixed to a distal end of the tip, a delivery sheath sleeved over the delivery inner shaft, a delivery handle configured to control axial movement of the delivery sheath, a first pre-embedded guidewire, a second pre-embedded guidewire, and a third pre-embedded guidewire;
a gap is defined between the delivery sheath and the delivery inner shaft for accommodating the fenestrated stent graft that is radially compressed;
the first pre-embedded guidewire is at least partially located outside the interventional device, at least partially extends through the delivery sheath, and at least partially extends out of a proximal end of the fenestrated stent graft;
the tubular covering membrane defines at least one fenestration, wherein the at least one fenestration comprises a second fenestration;
for a portion of the first pre-embedded guidewire that is located within the delivery sheath, part of the portion of the first pre-embedded guidewire that is on a distal side of the second fenestration is located outside the fenestrated stent graft, and part of the portion of the first pre-embedded guidewire that is adjacent to the proximal end of the fenestrated stent graft passes through the second fenestration and is located within the communication cavity of the fenestrated stent graft;
the fenestrated stent graft further comprises at least two embedded branch tubes; and
each of the second pre-embedded guidewire and the third pre-embedded guidewire is at least partially located outside the interventional device, the second pre-embedded guidewire at least partially extends through one of the at least two embedded branch tubes, and the third pre-embedded guidewire at least partially extends through another one of the at least two embedded branch tubes.

39. A method for suturing a fenestrated stent graft, wherein the method for suturing the fenestrated stent graft according to any one of claims 1 to 35 comprises:
disposing the enveloping band to surround the at least one fenestration, wherein the enveloping band comprises a first side edge and a second side edge opposite the first side edge, and the first side edge is closer to the at least one fenestration than the second side edge;
folding the first side edge to form the first region that has a multilayer membrane structure;
making the second side edge to form the second region that has at least one of a single-layer membrane structure or a multilayer membrane structure, wherein a number of membrane layers of the second region is less than a number of membrane layers of the first region; and
covering and surrounding at least a portion of the at least one fenestration with the first region;
suturing the first region to the fenestrated stent graft through a suture; and
suturing the second region to the fenestrated stent graft through a suture.
